# EUROPEAN PATENT APPLICATION

(11) **EP 1 621 556 A1**
(43) Date of publication of application: **01.02.2006**
(21) Application number: 04730018.1
(22) Date of filing: 28.04.2004
(51) Int. Cl.: C07K 19/00, C12N 15/62, C12P 21/02, C07K 14/195, C12N 15/31, C12N 1/21

(54) **METHOD OF PRODUCING TARGET PROTEIN, FUSED PROTEIN AND GENE THEREOF, PARTIAL SEQUENCE PROTEIN OF INTEIN AND GENE THEREOF, EXPRESSION VECTOR AND TRANSFORMANT**

(30) Priority: 28.04.2003 JP 2003124354; 28.04.2003 JP 2003124355; 18.12.2003 JP 2003421442; 18.12.2003 JP 2003421443
(71) Applicant: SEKISUI CHEMICAL CO., LTD., Osaka-shi, Osaka 530-8565 (JP)
(72) Inventor: TOGI, Akiko, Sekisui Chemical Co., Ltd., Mishima-gun, Osaka 6188589 (JP); FURUTANI, Masahiro, Sekisui Chemical Co., Ltd., Mishima-gun, Osaka 6188589 (JP)
(74) Representative: Grund, Martin
(86) International application number: PCT/JP2004/006191
(87) International publication number: WO 2004/096860

(57) **Abstract**

It is intended to provide techniques whereby a target protein is efficiently obtained as a normal protein even in the case of a protein which can be hardly expressed by usual recombinant DNA techniques. A fusion protein composed of a molecular chaperone, an intervening protein having an activity of cleaving a peptide bond, and a target protein is prepared, from which fusion protein the target protein is cleaved by the action of the intervening protein having the activity of cleaving a peptide bond. Examples of the intervening protein having the activity of cleaving a peptide bond include an intein and a protein consisting of a partial sequence of the intein.

## Description

### TECHNICAL FIELD

The present invention relates to a method of producing a target protein, a fusion protein and a gene thereof, a protein consisting of a partial sequence of an intein and a gene thereof, an expression vector, and a transformant. More particularly, it relates to a method of producing a target protein wherein the target protein is cleaved from a fusion protein composed of a molecular chaperone, an intervening protein having an activity of cleaving a peptide bond, and the target protein by the action of the intervening protein, the fusion protein and a gene thereof, the protein consisting of a partial sequence of an intein having the activity of cleaving a peptide bond and a gene thereof, an expression vector, and a transformant. According to the present invention, a target protein is efficiently obtained as a normal protein even in the case of a protein which can be hardly expressed by usual recombinant DNA techniques.

### BACKGROUND ART

Up to now, various protein expression systems including protein expression systems in many hosts such as bacteria, yeasts, insects, animal and plant cells, and transgenic animals and plants, and further cell-free translation systems have been established. Recently, genomes of various organisms have been continuously decoded, and needs to produce recombinant proteins from genes and analyze them are increasing more. However, not all of target proteins can be readily obtained by these protein expression systems. It is still difficult to obtain recombinant proteins, for example, in the case of proteins that are toxic to a host or proteins that tend to be insoluble. More specifically, if a target protein is toxic to a host, synthesis of the protein in the host is inhibited, resulting in decrease of the expression level. Further, even if the target protein is expressed, only an abnormal protein having an incorrect stereostructure may be obtained due to abnormal folding of the target protein. Especially, the abnormal protein often forms an insoluble aggregate called an inclusion body. It is so difficult to refold the abnormal protein that has once formed the inclusion body to make a normal protein, and it often ends in failure.

In order to prevent a target protein from forming an inclusion body, a method of expressing the protein as a fusion protein with a glutathione-S-transferase (GST), a thioredoxin, a maltose-binding protein, or the like is proposed. However, it is difficult to suppress the formation of the inclusion body at high efficiency (for example, Smith, D. B., et al., Gene 67, 31-40, 1988). Alternatively, there is also an attempt to express a target protein as a normal protein having a correct stereostructure by the action of a molecular chaperon that is a protein assisting a protein-folding reaction. For example, a target protein is co-expressed in a host with a molecular chaperone, whereby the target protein is expressed as a normal protein. However, even this method can not achieve a remarkable increase in the amount of the target protein obtained as the normal protein (Nishihara et al., Applied and environmental microbiology, 64, 1694-1699, 1998).

As another method to use the action of a molecular chaperone, it is also attempted to use a chaperonin subunit linkage wherein subunits of a chaperonin that is a kind of the molecular chaperone are linked to one another. More specifically, a fusion protein including the target protein and a chaperonin subunit linkage composed of 1 to 20 chaperonin subunits serially linked to one another is prepared to be used. At this time, the target protein is accommodated in the stereostructure of the chaperonin in the same way it forms a complex with a native chaperonin. As a consequence, the target protein is readily obtained as a normal protein that is normally folded, thereby ensuring to prevent formation of an inclusion body. Further, even if the target protein is toxic to a host; its accommodation in the chaperonin prevents the toxicity. Still further, the target protein is protected from an attack by proteases derived from the host (see WO02/052029 A1). Specifically, this method can express proteins being toxic to a host such as various virus antigens, various antibodies, seven transmembrane receptors (G-protein coupled receptors), or cytokines.

This method requires cleavage of a target protein from a fusion protein as well as methods used for conventional fusion proteins such as a GST, a thioredoxin, or a maltose-binding protein. Specifically, a restriction protease acts on a fusion protein, thereby cleaving a target protein from the fusion protein. More specifically, a restriction protease recognition sequence is arranged in advance between a target protein and a chaperonin subunit linkage to cleave the target protein by the action of the protease. Herein, restriction proteases include thrombin, FXa (activated blood coagulation factor X), enterokinase, and PreScission protease.

However, in this method, the target protein itself may be decomposed by the protease, depending on the nature of the target protein. For example, when the target protein is a hepatitis C virus antigen, its high hydrophobicity reduces the stringency of recognition by a restriction protease, resulting in cleaving other parts as well as the recognition sequence, with a consequence that the hepatitis C virus antigen itself is decomposed. Other proteins having a high hydrophobic region, a transmembrane region, or the like have also the same problem.

Cleavage efficiency of proteases varies according to the type of a target protein. Consequently, proteases may not act and thus a target protein may not be cleaved, depending on the type of the target protein. The difference of the cleavage efficiency is attributed to the ability to penetrate thereinto and/or to approach of the restriction protease when the protease approaches a cleavage site to cleave the recognition sequence. Further, in this method, when the cleaved target protein is purified, it is necessary to remove the added restriction protease. Consequently, this method has also such a problem as making purification of a target protein cumbersome and complicated.

### DISCLOSURE OF THE INVENTION

It is an object of the present invention to provide a fusion protein composed of a molecular chaperone, an intervening protein having an activity of cleaving a peptide bond, and a target protein and a gene thereof, a method of producing a target protein wherein the target protein is cleaved from the fusion protein by the action of the intervening protein, a protein consisting of a partial sequence of an intein having an activity of cleaving a peptide bond and a gene thereof, an expression vector, and a transformant.

As the result of diligent researches, the inventors have found that combination of a protein having an activity of cleaving a peptide bond as typified by an intein and a molecular chaperon produces a protein as a normal protein efficiently even if the protein is hardly expressed by means of usual recombinant DNA techniques. Further, a fusion protein, which is useful for the producing method, composed of a molecular chaperone, an intervening protein having an activity of cleaving a peptide bond, and a target protein is isolated. Still further, a protein consisting of a partial sequence of an intein having an activity of cleaving a peptide bond is isolated. Yet further, a gene encoding them is isolated, and an expression vector containing the gene and a transformant containing the expression vector are isolated, so as to accomplish the present invention.

More specifically, a summary of the present invention is as follows:
(1) A fusion protein including an intervening protein having an activity of cleaving a peptide bond, a molecular chaperone, and a target protein, wherein the intervening protein has one terminus to which either the molecular chaperone or its subunit is linked with a peptide bond, and wherein the intervening protein has the other terminus to which the target protein is linked with a peptide bond;
(2) The fusion protein as described in (1) wherein the molecular chaperone is a chaperonin composed of a plurality of chaperonin subunits;
(3) The fusion protein as described in (2) wherein the intervening protein having an activity of cleaving a peptide bond is linked to either an independent chaperonin subunit or a chaperonin subunit linkage, the chaperonin subunit linkage being composed of 2 to 10 chaperonin subunits serially linked to one another with a peptide bond;
(4) The fusion protein as described in (3) wherein the intervening protein having an activity of cleaving a peptide bond is linked to at least one site selected from a group consisting of the N-terminus of either the independent chaperonin subunit or the chaperonin subunit linkage, the C-terminus of either the independent chaperonin subunit or the chaperonin subunit linkage, and a linking site of the chaperonin subunits of the chaperonin subunit linkage;
(5) The fusion protein as described in (3) or (4) wherein the ratio of the number of the chaperonin subunits to the number of the target protein is in the range of from 1 : 1 to 10 : 1;
(6) The fusion protein as described in (1) wherein the molecular chaperone is a peptidyl prolyl cis-trans isomerase;
(7) The fusion protein as described in (6) wherein the intervening protein having an activity of cleaving a peptide bond is linked to the N-terminus and/or the C-terminus of the peptidyl prolyl cis-trans isomerase;
(8) The fusion protein as described in one of (1) to (7) wherein the molecular chaperone is derived from one selected from a group consisting of bacteria, archaea, and eukaryotes;
(9) The fusion protein as described in one of (1) to (8) wherein the intervening protein having an activity of cleaving a peptide bond is either an intein or a protein consisting of a partial sequence of an intein;
(10) The fusion protein as described in (9), the intein cleaving only the N-terminus, but not the C-terminus;
(11) The fusion protein as described in (9), the intein cleaving only the C-terminus, but not the N-terminus;
(12) The fusion protein as described in (9), the protein consisting of a partial sequence of an intein including 20 to 120 amino acid residues from the C-terminus of the intein;
(13) The fusion protein as described in one of (9) to (12), the intein being derived from Synechocystis sp.;
(14) The fusion protein as described in one of (9) to (12), the intein being derived from one selected from a group consisting of Mycobacterium xenopi, Saccharomyces cerevisiae, and Halobacterium sp.;
(15) The fusion protein as described in (12), the protein consisting of a partial sequence of an intein being either a protein having an amino acid sequence as shown in SEQ ID No. 1, or a protein having an amino acid sequence wherein at least one amino acid is deleted, substituted, added, or inserted and having an activity of cleaving a peptide bond;
(16) The fusion protein as described in (12), the protein consisting of a partial sequence of an intein having homology of at least 50% or more to an amino acid sequence as shown in SEQ ID No. 1 and having an activity of cleaving a peptide bond;
(17) The fusion protein as described in one of (1) to (16) wherein the intervening protein having an activity of cleaving a peptide bond is a protease;
(18) An isolated gene encoding the fusion protein as described in one of (1) to (17);
(19) A method of producing a target protein, the method including a step of cleaving the target protein from the fusion protein as described in one of (1) to (17) by the action of the intervening protein having an activity of cleaving a peptide bond;
(20) A method of producing a target protein, the method including steps of preparing the fusion protein as described in one of (1) to (17), inducing an activity of cleaving a peptide bond of the intervening protein having the activity contained in the fusion protein prepared in the precedent step, and isolating the target protein cleaved from the fusion protein by cleaving a part of the fusion protein by the action of the intervening protein having the activity of cleaving a peptide bond induced in the precedent step;
(21) The method of producing the target protein as described in (20) wherein the activity of cleaving a peptide bond is induced by exposure of the fusion protein at a temperature of 20 to 37°C;
(22) The method as described in (20) or (21) wherein the activity of cleaving a peptide bond is induced by exposure of the fusion protein at a hydrogen ion concentration of pH 6 to 8;
(23) The method as described in one of (20) to (22), wherein the activity of cleaving a peptide bond is induced by addition of a thiol;
(24) The method as described in (19), the method including steps of preparing an expression vector having a gene encoding the fusion protein introduced, introducing the expression vector obtained in the precedent step into a host so as to express the fusion protein, and cleaving a target protein from the fusion protein obtained in the precedent step;
(25) The method as described in (19), the method including steps of introducing genes encoding the fusion protein into two different plasmids capable of coexistence and replication in the same host so as to produce two kinds of expression vectors, introducing the two kinds of expression vectors obtained in the precedent step into the same host so as to express the fusion protein, and cleaving a target protein from the fusion protein obtained in the precedent step by the action of the intervening protein having an activity of cleaving a peptide bond;
(26) The method as described in (19), the method including steps of introducing a gene encoding the fusion protein into one of two different plasmids capable of coexistence and replication in the same host and a gene encoding only a molecular chaperone into the other of the plasmids so as to produce two kinds of expression vectors, introducing the two kinds of expression vectors obtained in the precedent step into the same host so as to express the fusion protein and the molecular chaperone, and cleaving a target protein from the fusion protein obtained in the precedent step by the action of the intervening protein having an activity of cleaving a peptide bond;
(27) The method as described in one of (24) to (26), the host being one selected from a group consisting of bacteria, yeasts, animal cells, plant cells, insect cells, animals, plants, and insects;
(28) The method as described in one of (19) to (26) wherein the fusion protein is expressed in the host, the host being one selected from a group consisting of bacteria, yeasts, animal cells, plant cells, insect cells, animals, plants, and insects;
(29) The method as described in (19) being performed in a cell-free translation system;
(30) The method as described in one of (19) to (29) wherein the molecular chaperone is a chaperonin forming a chaperonin ring constituted by five to ten chaperonin subunits assembled in a ring, and wherein the target protein is accommodated in the chaperonin ring;
(31) A protein consisting of a partial sequence of an intein composed of 20 to 120 amino acid residues from the C-terminus of the intein;
(32) The protein consisting of a partial sequence of an intein as described in (31) wherein the intein is derived from Synechocystis sp.;
(33) The protein consisting of a partial sequence of an intein as described in (31) wherein the intein is derived from one selected from a group consisting of Mycobacterium xenopi, Saccharomyces cerevisiae, and Halobacterium sp.;
(34) The protein consisting of a partial sequence of an intein as described in (31), being either a protein having an amino acid sequence as shown in SEQ ID No. 1, or a protein having an amino acid sequence wherein at least one amino acid is deleted, substituted, added, or inserted and having an activity of cleaving a peptide bond;
(35) The protein consisting of a partial sequence of an intein as described in (31) having homology of at least 50% or more to an amino acid sequence as shown in SEQ ID No. 1, and having an activity of cleaving a peptide bond;
(36) An isolated gene encoding the protein consisting of a partial sequence of an intein as described in one of (31) to (35);
(37) An expression vector containing the gene as described in (18) or (36);
(38) A transformant containing the expression vector as described in (37);
(39) A method of producing a target protein, the method including steps of a first step of producing a first fusion protein composed of a protein consisting of a partial sequence of an intein, either a molecular chaperone or its subunit linked to one terminus of the protein consisting of a partial sequence of an intein with a peptide bond, and a first precursor of target protein linked to the other terminus of the protein consisting of a partial sequence of an intein with a peptide bond, a second step of producing a second fusion protein composed of a protein consisting of a remaining partial sequence of the intein, either a molecular chaperone or its subunit linked to one terminus of the protein consisting of a remaining partial sequence of an intein with a peptide bond, and a second precursor of target protein linked to the other terminus of the protein consisting of a remaining partial sequence of an intein with a peptide bond, a third step of cleaving the first precursor of target protein from the first fusion protein obtained in the first step and further cleaving the second precursor of target protein from the second fusion protein obtained in the second step, both by a cleaving function of the protein consisting of a partial sequence of an intein and the protein consisting of a remaining partial sequence of the intein, and a fourth step of assembling the first precursor of target protein and the second precursor of target protein both obtained in the third step by an assembling function of the protein consisting of a partial sequence of an intein and the protein consisting of a remaining partial sequence of the intein;
(40) A method of producing a target protein, the method including steps of cleaving a first precursor and a second precursor of target protein from a fusion protein composed of a molecular chaperone, a protein consisting of a partial sequence of an intein linked to one terminus of the molecular chaperone and a protein consisting of a remaining partial sequence of the intein linked to the other terminus of the molecular chaperone both with a peptide bond, and further the first precursor of target protein linked to the protein consisting of a partial sequence of an intein and the second precursor of target protein linked to the protein consisting of a remaining sequence of the intein both with a peptide bond by a cleaving function of the proteins consisting of a partial sequence of intein and the protein consisting of a remaining partial sequence of the intein, and assembling the first precursor of target protein and the second precursor of target protein by an assembling function of the protein consisting of a partial sequence of an intein and the protein consisting of a remaining partial sequence of the intein;
(41) The method as described in (39) or (40) wherein the fusion protein is expressed in a host;
(42) The method as described in (41), the host being one selected from a group consisting of bacteria, yeasts, animal cells, plant cells, insect cells, animals, plants, and insects; and
(43) The method as described in (39) or (40) being performed in a cell-free translation system.

According to the method of producing a target protein in the present invention, the target protein is obtained as a normal protein efficiently, even if the target protein is hardly expressed by means of usual recombinant DNA techniques. Further, the target protein is cleaved from a fusion protein more simply and more safely.

According to the fusion protein in the present invention, a target protein is expressed as a as a part of the fusion protein, even if the target protein is hardly expressed by means of usual recombinant DNA techniques. Further, the target protein is cleaved more simply and more safely by the action of an intervening protein having an activity of cleaving a peptide bond contained in the fusion protein.

According to the protein consisting of a partial sequence of an intein in the present invention, a molecular weight of a fusion protein composed of a molecular chaperone and a target protein is lessened because of its less molecular weight than that of a native intein. Consequently, the fusion protein is expressed more readily by means of recombinant DNA techniques.

According to the gene of a protein consisting of a partial sequence of an intein in the present invention, a protein consisting of a partial sequence of an intein is expressed by recombinant DNA techniques.

According to the vector in the present invention, a gene encoding a fusion protein composed of a molecular chaperone, an intervening protein having an activity of cleaving a peptide bond, and a target protein is produced only if a gene of the target protein is introduced, thereby readily introducing the gene of the fusion protein into a host. Further, according to the vector of the invention, a gene of a protein consisting of a partial sequence of an intein is readily introduced into a host.

According to the transformant in the present invention, a fusion protein composed of a molecular chaperone, an intervening protein having an activity of cleaving a peptide bond, and a target protein is produced. Further, the use of the transformant of the invention produces a protein consisting of a partial sequence of an intein.

### BRIEF DESCRIPTION OF THE DRAWINGS

Fig. 1 is an illustration showing a constitution of a main part of an expression vector pETDH(TCPβ)₄I. In the figure, (TCPβ)₄ denotes a gene encoding a chaperonin β subunit 4-times linkage, SspI denotes a gene encoding an intein, 6His denotes a gene encoding six histidine residues, and TC denotes a termination codon;
Fig. 2 is a photograph showing a result of Western blot analysis of supernatants of cell lysates obtained in Example 1 and Comparative Example 1, using an antibody recognizing an antibody light chain;
Fig. 3A is a photograph showing a result of Western blot analysis of supernatants of cell lysates obtained in Example 2, using an antibody recognizing a hepatitis C virus core antigen;
Fig. 3B is a photograph showing a result of Western blot analysis of supernatants of cell lysates obtained in Comparative Example 2, using an antibody recognizing a hepatitis C virus core antigen;
Fig. 4 is an illustration showing a constitution of a main part of an expression vector pT (GroEL)₇I. In the figure, (GroEL)₇ denotes a gene encoding a GroEL subunit 7-times linkage, NSspI denotes a gene encoding an intein sequence modified so as to cleave only the N-terminus, FLAG denotes a gene encoding FLAG peptide, and TC denotes a termination codon;
Fig. 5 is a photograph showing a result of Western blot analysis of supernatants of cell lysates obtained in Example 3 and Comparative Example 3 (1: the supernatant of cell lysate in Comparative Example 3, 2: the supernatant of cell lysate in Example 3), using an anti-FLAG peptide antibody;
Fig. 6 is an illustration showing a constitution of a main part of an expression vector pET TPPIase I. In the figure, TPPIase denotes a gene encoding a TPPIase sequence, SspI denotes a gene encoding an intein, 6His denotes a gene encoding six histidine residues, and TC denotes a termination codon;
Fig. 7 is a photograph showing a result of Western blot analysis of supernatants of cell lysates obtained in Example 4 and Comparative Example 4 (1: the supernatant of cell lysate in Example 4, 2: the supernatant of cell lysate in Comparative Example 4, 3: GFP-6His standard), using an antibody recognizing a histidine;
Fig. 8 is a photograph showing a result of Western blot analysis of supernatants of cell lysates obtained in Example 5 and Comparative Example 5 (1: a control using a vector without an SspI gene, 2: the supernatant of cell lysate in Example 5, 2: the supernatant of cell lysate in Comparative Example 5);
Fig. 9 is a photograph showing a result of SDS-PAGE analysis with CBB straining in Example 5 and Comparative Example 5 (1: the supernatant of cell lysate in Comparative Example 5, 2: FLAG column-purified fractions of the supernatant of cell lysate in Comparative Example 5, 3: a control using a vector without SspI gene, 4: FLAG column-purified fractions of the control using the vector without SspI gene);
Fig. 10 is a photograph showing a result of Western blot analysis in Example 6 and Comparative Example 6 (1: the supernatant of cell lysate in Comparative Example 6, 2: the supernatant of cell lysate in Example 6);
Fig. 11 is a photograph showing a result of Western blot analysis in Example 7 and Comparative Example 7 (1: GFP-6His standard, 2: the supernatant of cell lysate in Example 7, 2: the supernatant of cell lysate in Comparative Example 7);
Fig. 12 is an illustration showing a constitution of a main part of an expression vector pETDH(TCPβ)₄I-2. In the figure, (TCPβ)₄ denotes a gene encoding a chaperonin β subunit 4-times linkage, SspI denotes a gene encoding an intein, 6His denotes a gene encoding six histidine residues, and TC denotes a termination codon;
Fig. 13 is an illustration showing a constitution of a main part of an expression vector pETDH(TCPβ)₄MMI-2. In the figure, (TCPβ)₄ denotes a gene encoding a chaperonin β subunit 4-times linkage, SspI denotes a gene encoding an intein, 6His denotes a gene encoding six histidine residues, and TC denotes a termination codon;
Fig. 14 is a photograph showing a result of Western blot analysis of supernatants of cell lysates obtained in Example 8 and Comparative Example 8 (1: the supernatant of cell lysate in Example 8 wherein the cell has pETDH(TCPβ)₄MMI·AbL-2 introduced therein, 2: the supernatant of cell lysate in Comparative Example 8 wherein the cell has pETDH(TCPβ)₄I·AbL-2 introduced therein, 3: Comparative Example 8);
Fig. 15A is a photograph showing a result of SDS-PAGE analysis with CBB straining of a supernatant of cell lysate obtained in Example 9 (1: the supernatant of cell lysate in Example 9);
Fig. 15B is a photograph showing a result of SDS-PAGE analysis with CBB straining of a supernatant of cell lysate obtained in Comparative Example 9 (2: the supernatant of cell lysate in Comparative Example 9, 3: a supernatant of host cell lysate;
Fig. 16 is a photograph showing a result of Western blot analysis in Example 9 (1: a supernatant of cell lysate in Example 9, 2: a supernatant of host cell lysate;
Fig. 17 is an illustration showing a constitution of a main part of an expression vector pGEX MMI. In the figure, GST denotes a gene encoding a glutathione-S-transferase, MMI denotes a gene encoding an MMI, 6His denotes a gene encoding six histidine residues, and TC denotes a termination codon;
Fig. 18 is a photograph showing a result of Western blot analysis of supernatants of cell lysates obtained in Example 10 and Comparative Example 10 (1: the supernatant of cell lysate in Example 10-1, 2: the supernatant of cell lysate in Example 10-2, 3: the supernatant of cell lysate in Comparative Example 10);
Fig. 19 is an illustration showing a constitution of a main part of an expression vector pET TPPIase MMI. In the figure, TPPIase denotes a gene encoding TPPIase, MMI denotes a gene encoding an MMI, 6His denotes a gene encoding six histidine residues, and TC denotes a termination codon;
Fig. 20 is a photograph showing a result of Western blot analysis in Example 11 and Comparative Example 11 (1: GFP-6His standard, 2: a supernatant of cell lysate in Comparative Example 11-1, 3: a supernatant of cell lysate in Example 11, 4: a supernatant of cell lysate in Comparative Example 11-2).

### DESCRIPTION OF THE PREFERRED EMBODIMENTS

A fusion protein in the present invention is composed of an intervening protein having an activity of cleaving a peptide bond, a molecular chaperone, and a target protein. The use of the fusion protein in the invention cleaves the target protein from the fusion protein by the action of the intervening protein having an activity of cleaving a peptide bond, thereby obtaining the target protein.

There are two kinds of an intervening protein having an activity of cleaving a peptide bond, one acting on other proteins to cleave a peptide bond of the proteins like proteases, one acting itself to autocatalytically cleave a peptide bond like inteins. The intervening protein contained in the fusion protein in the invention includes a protease, an intein, a hedgehog protein, and a self-splicing protein.

An intein is a protein performing protein splicing that has been found in all three domains of life (eukaryotes, archaea, and bacteria). In the case of the intervening protein having an activity of cleaving a peptide bond contained in the fusion protein in the present invention being an intein, the fusion protein is cleaved by an autocatalytic action of the intein to cleave the target protein. A native intein has an activity of cleaving a peptide bond of the N-terminus and the C-terminus of itself and further has an activity of binding the cleaved termini with a peptide bond. The intein contained in the fusion protein in the invention is preferably a modified intein that has only an activity of cleaving a peptide bond and deletes an activity of binding the cleaved termini. More specifically, this modified intein inhibits rejoining of a target protein and a molecular chaperone, thereby cleaving the target protein from the fusion protein more readily. A modified intein is obtained by substitution of amino acid residues that is a part of a native intein. A modified intein having only an activity of cleaving the C-terminus (hereinafter referred to as a "C-intein") without an activity of cleaving the N-terminus is obtained by modifying a cysteine of the N-terminus of a native intein to an alanine. A modified intein having only an activity of cleaving the N-terminus (hereinafter referred to as an "N-intein") without an activity of cleaving of C-terminus is obtained by modifying an asparagine to an alanine (Mathys S et al., Gene, 231, 1-13, 1999). These types of modified intein do not cleave both of the termini, so as never to rejoin a target protein and a molecular chaperone.

The intein included in the fusion protein in the present invention is derived from such as Synechocystis sp. (cyanobacteria Synechocystis sp. PCC 6803 strain DnaB helicase intein, for example), Mycobacterium xenopi (Mycobacterium xenopi Gyrase A intein, for example), and Saccharomyces cerevisiae (Saccharomyces cerevisiae Vacuolar Membrane ATPase intein). Especially, Synechocystis sp. PCC 6803 strain DnaB helicase intein holds a cleaving activity, even if being modified so as to be composed of the N-terminus domain and the C-terminus domain without a central domain having an endonuclease activity. Consequently, the intein modified in this way has less molecular weight than other derived inteins, so as to have an advantage in expression by means of recombinant DNA techniques. As long as having an activity of cleaving a peptide bond, an intein not involved herein is also adoptable as an intervening protein having an activity of cleaving a peptide bond contained in the fusion protein in the present invention.

The nucleotide sequence of a gene encoding a C-intein obtained by removal of the endonuclease region of Synechocystis sp. strain PCC 6803 DnaB helicase intein (hereinafter referred to as an "SspI") and by substitution of the amino acid of the N-terminus thereof to an alanine and the amino acid sequence corresponding thereto are shown in SEQ ID No. 3, and only the amino acid sequence corresponding thereto is shown in SEQ ID No. 4.

Further, in the fusion protein in the present invention, the intervening protein having an activity of cleaving a peptide bond may be not the full-length intein but a protein consisting of a partial sequence of an intein having the same activity as the full-length intein. Proteins consisting of a partial sequence of an intein in the fusion protein in the invention are selected from a group of proteins consisting of 20 to 120 amino acid residues from the C-terminus of the intein. These proteins each consisting of a partial sequence of an intein have the same protein-splicing function and the same activity of cleaving a peptide bond of the C-terminus of itself as the full-length intein.

A protein, for example, consisting of 20 to 120 amino acid residues from the C-terminus of the amino acid sequences of an SspI shown in SEQ ID No. 4 has every function of the full-length of the SspI, being applied to the fusion protein in the invention. More preferably, a protein consisting of 48 amino acid residues from the C-terminus of the amino acid sequences of the SspI shown in SEQ ID No. 4 (hereinafter referred to as an "MMI") is a suitable component for the fusion protein in the invention. The amino acid sequence of the MMI is shown in SEQ ID No. 32. Still further, a protein having an amino acid sequence wherein at least one amino acid of the amino acid sequence shown in SEQ ID No. 32 is deleted, substituted, added, or inserted and having an activity of cleaving a peptide bond, that is, the substantially same protein as the MMI is a suitable component for the fusion protein in the invention. Yet further, a protein having homology of at least 60% to the amino acid sequence shown in SEQ ID No. 32, preferably one having homology of 80% or more thereto, more preferably one having homology of 90% or more thereto, or further preferably one having homology of 95% or more thereto, and having an activity of cleaving a peptide bond may be a component of the fusion protein in the invention. Further, a protein consisting of a partial sequence of an intein other than the examples as just described may be a component of the fusion protein in the invention as long as it has an activity of cleaving a peptide bond.

A gene encoding the MMI described above includes isolated DNA having a nucleotide sequence shown in SEQ ID No. 31. Further, DNA being hybridizable with the DNA shown in SEQ ID No. 31 under stringent conditions and encoding a protein having an activity of cleaving a peptide bond encodes the substantially same protein as the MMI. Herein, hybridizable DNA is one detected by means of colony hybridization method, plaque hybridization method, Southern blot hybridization method, or the like using DNA as a probe. Stringent conditions are those of 0.1xSSC solution (1×SSC solution is composed of 150 mM sodium chloride and 15 mM sodium citrate), 1% SDS at 65°C for 24 hours. DNA to be hybridized under these conditions is substantially the same DNA as the MMI gene shown in SEQ ID No. 31. Herein, hybridization is performed according to methods described in "Molecular Cloning" Second Edition, "Current Protocols in Molecular Biology", DNA Cloning 1: Core Techniques, A practical Approach, Second Edition, Oxford University (1995) and the like.

Whether hybridizable or not under stringent conditions, products by DNA having homology of at least 60% to the nucleotide sequence shown in SEQ ID No. 31, preferably having homology of 80 % or more thereto, DNA more preferably having homology of 90% or more thereto, or still more preferably having homology of 95% or more thereto, and encoding a protein having an activity of cleaving a peptide bond may be components of the fusion protein of the invention.

When an intervening protein having an activity of cleaving a peptide bond is other than an intein, in the case of a protease, for example, an amino acid sequence to be recognized by the protease may be inserted between the protease and a target protein so that the fusion protein is cleaved by the action of the protease. A hedgehog protein, which is an extracellular secretory signal transfer factor of Drosophila and has an activity of cleaving a peptide bond for its own processing, may be a component of the fusion protein of the invention. As for a protease or a hedgehog protein, not only native proteins having their full length but also proteins consisting of a partial sequence thereof and having the same activity may be also a component of the fusion protein in the invention. In the present invention, the terms such as a protease and a hedgehog protein include proteins consisting of a partial sequence thereof and having the same activity as native proteins having their full length.

A target protein of which the fusion protein in the invention is composed is not particularly limited and may be any useful protein such as disease-related gene products derived from higher animals such as humans and mice and enzyme groups useful in chemical processes. Examples thereof include proteins such as coat protein, core protein, protease, reverse transcriptase, and integrase encoded by a genome of pathogenic viruses such as a hepatitis B virus, a hepatitis C virus, an HIV, an influenza virus, or the like. Other examples thereof include heavy chains in antibodies derived from mammalians, light chains in antibodies derived from mammalians, constant region in antibodies derived from mammalians, the full-length of Fv region single-chain antibodies (scFv) in antibodies derived from mammalians or a partial protein of six or more residues thereof, Fab (antigen binding fragments), (Fab')₂ fragments, CH1-CH2-CH3 fragments, CH1-CH2 fragments, CH2-CH3 fragments, CH1 fragments, CH2 fragments, CH3 fragments, CL fragments, and whole antibody for therapeutic and diagnostic use. Still other examples include seven transmembrane receptor protein (G protein-coupled receptor), growth factors such as platelet growth factor, hematopoietic stem cell growth factor, hepatocyte growth factor, transforming growth factor, nerve growth-trophic factor, fibroblast growth factor and insulin-like growth factor; and cytokines such as tumor necrosis factor, interferon, erythropoietin, granulocyte-colony simulating factor, macrophage-colony stimulating factor, granulocyte macrophage-colony stimulating factor, interleukins such as interleukin 3 and interleukin 5, albumin, and human growth hormone. Yet other examples thereof include Green Fluorescent Protein (hereinafter referred to as a "GFP") available for fluorescein labeling of a functional protein in cells in order to examine phenomenon in living cells. Further, an artificial protein in which two or more proteins selected from these proteins are linked may be applicable as a target protein of which the fusion protein of the invention is composed.

A molecular chaperone or its subunit of which the fusion protein in the invention is composed is not particularly limited, and as long as having a function of assisting protein folding, any kind thereof is applicable. Its examples include a chaperonin complex consisting of a plurality of chaperonin subunits and a peptidyl prolyl cis-trans isomerase (hereinafter referred to as a "PPIase").

Herein, there are two kinds of molecular chaperones, one functioning in a single molecule, and one forming a complex consisting of a plurality of subunits. In the case of the former molecular chaperon contained in the fusion protein in the invention, the single molecular chaperone is linked to the intervening protein. On the other hand, in the case of the latter molecular chaperon contained in the fusion protein in the invention, the subunits are linked to the intervening protein. A PPIase is an example of the former one and a chaperonin is an example of the latter one.

A chaperonin is a complex composed of about 1 to 20 subunits (chaperonin subunits) with a molecular weight of about 60 kDa. It is known that a chaperonin is a kind of a heat-shock protein induced by adding stress such as a heat shock to cells. A chaperonin is found in every living thing such as bacteria (Escherichia coli, for example), archaea (Thermococcus sp., Pyrococcus horikoshii, Aeropyrum pernix, and the like, for example), and eukaryotes (yeasts, mice, humans, and the like, for example), and has functions of assisting protein folding and inhibiting denaturation.

It is also known that an artificial protein wherein a plurality of chaperonin subunits are serially linked to one another via a peptide bond (hereinafter referred to as a "chaperonin subunit linkage") forms a complex as well as a native chaperonin. At this time, it is known that about 2 to 10 chaperonin subunits have a linear or a ring structure (hereinafter referred to as a "chaperonin ring") as well as a native chaperonin. Herein, in contrast with the term "chaperonin subunit linkage", a chaperonin subunit to which another chaperonin subunit is not linked via a peptide bond is referred to as an "independent chaperonin subunit."

In the fusion protein in the present invention, a target protein is linked via an intervening protein having an activity of cleaving a peptide bond to at least one site selected from a group consisting of the N-terminus of an independent chaperonin subunit or a chaperonin subunit linkage, the C-terminus of an independent chaperonin subunit or a chaperonin subunit linkage, and a linking site of chaperonin subunits of a chaperonin subunit linkage. In each case, the site to which the target protein is linked is preferably selected so that the target protein is accommodated in a cavity formed in a plurality of chaperonin subunits, that is, at least one-layer chaperonin ring.

A chaperonin forming a two-layer ring structure consisting of 10 to 20 chaperonin subunits, that is, a two-layer structure wherein two chaperonin rings non-covalently associated on each other's ring plane (hereinafter also referred to a two-layer ring structure as a chaperonin complex) is suitable for use in an example of the molecular chaperone of which the fusion protein in the invention is composed. An Escherichia coli chaperonin GroEL having a cavity with an inner diameter of 4.5 nm and a height of 14.5 nm, for example, is suitable. When a chaperonin is used as a molecular chaperone, a fusion protein is preferably composed so that a target protein is accommodated in the chaperonin ring. Herein a bacterial or an archaeal chaperonin is especially suitable to produce the fusion protein in the invention because of its easy expression in a host.

As for the chaperonin described above, not only a wild type but also an amino acid mutant is available as long as it maintains a self-assembling ability into a ring structure. The use of a mutant in which an association ability of each chaperonin subunit is lowered, for example, facilitates a collection of the accommodated target protein. The use of bacteria as a host to express a chaperonin often increases an amount of the chaperonin expressed if a chaperonin subunit derived from the same bacteria as the host is expressed. Consequently, production efficiency of a target protein is often increased.

The number of chaperonin subunits constituting a chaperonin is varied depending on the living thing from which the chaperonin is derived. For example, the number of chaperonin subunits is usually seven in the case of a chaperonin derived from bacteria, while the number of that is usually eight to nine in the case of a chaperonin derived from archaea, while the number of that is usually eight in the case of the chaperonin derived from eukaryotes. Consequently, the number of chaperonin subunits of a chaperonin contained in the fusion protein in the present invention is selected the optimum number depending on its derivation.

In the fusion protein in the present invention using a chaperonin as a molecular chaperone, the ratio of the number of chaperonin subunits forming a ring structure to the number of a target protein in the fusion proteins is usually in the range of from 1 : 1 to 10 : 1, preferably in the range of from 1 : 1 to 9 : 1. If and when the ratio of the number of the chaperonin subunit is bigger than 10 : 1, a substantial amount of production of a target protein expressed may be reduced, and as well result in difficulty of formation of a ring structure of a chaperonin.

More specifically, when a bacterial chaperonin wherein the number of chaperonin subunits is seven is used, the ratio of the number of chaperonin subunits : the number of a target protein is preferably 1 : 1 or 7 : 1 for easy formation of a structure of a chaperonin complex, and when an archaeal chaperonin wherein the number of chaperonin subunits is eight is used, the number of chaperonin subunits : the number of a target protein is preferably 1 : 1, 2 : 1, 4 : 1, or 8 : 1 for easy formation of a structure of a chaperonin complex.

A PPIase is another example of a molecular chaperone. A PPIase is one of protein folding factors involved in folding a protein, and has an activity of catalyzing a cis-trans isomerization reaction of the N-terminus peptide bond of a proline residue (PPIase activity) among amino acids in a target protein during protein folding in a cell.

PPIases are classified into three types, an FK506 Binding Protein type (FKBP type), a cyclophilin type, a parvulin type based on sensitivity to its inhibitor. An FKBP-type PPIase is a PPIase, an activity of which is inhibited by FK506 that is one of immunosuppressive agents, and homologues thereof. A Cyclophilin-type PPIase is a PPIase having sensitivity to cyclosporin that is another immunosuppressive agent, and homologues thereof. On the other hand, a parvulin-type PPIase is a PPIase exhibiting no sensitivity to those immunosuppressive agents, and having an activity being inhibited by juglone. These three types of PPIases have little homology on an amino acid primary sequence.

A PPIase contained in the fusion protein in the present invention is not particularly limited, and may be any type of PPIase among the three types of PPIases described above. Examples of the FKBP-type PPIase include archaeal FKBP-type PPIases, trigger factor-type PPIases (Huang, Protein Sci., 9, 1254-, 2000), FkpA-type PPIases (Arie, Mol. Microbiol., 39, 199-, 2001), and FKBP52-type PPIases (Bose, Science, 274, 1715-, 1996). Examples of the cyclophilin-type PPIase include CyP40-type PPIase (Pirkl, J. Mol. Biol., 308, 795-, 2001). Examples of the parvulin-type PPIase include SurA-type PPIases (Behrens, EMBO J., 20, 285-, 2001). In the present invention, these PPIases include substantially an equivalent polypeptide having a vary similar amino acid sequence and having a similar action, a polypeptide containing at least a part of these PPIases and having a similar action, and a polypeptide containing a part of an amino acid of these PPIases modified to another amino acid and having a similar action.

Regarding a function of the archaeal FKBP-type PPIase, interestingly, it has been found that it has not only a PPIase activity, but also a molecular chaperone activity of inhibiting irreversible aggregation of a protein and, at the same time, promoting refolding of a denatured protein (Furutani, Biochemistry, 39, 453-, 2000; Ideno, Eur. J. Biochem., 267, 3139-, 2000; Ideno, Biochem. J., 357, 465-, 2001; Ideno, Appl. Env. Microbiol., 68, 464-, 2002). A molecular chaperone activity is originally an activity which was found in protein folding system of a chaperonin known one of molecular chaperones or of DnaK/DnaJ/GrpE system. These exert a function of supporting so that a polypeptide biosynthesized in a cell is folded into a correct form. Thereupon, hydrolysis of a high energy substance such as ATP is required, whereas the archaeal FKBP-type PPIase is different in that a reaction of hydrolyzing the high energy substance is not required upon exertion of the molecular chaperone activity. As a PPIase contained in the fusion protein in the present invention, an archaeal FKBP-type PPIase is particularly suitable since it folds a target protein more correctly than a PPIase without a molecular chaperone activity.

As a PPIase having a molecular chaperone activity used in the present invention, a PPIase other than the PPIase exemplified above may be suitably used as long as it is a PPIase having an equivalent molecular chaperone activity.

In the method of producing a target protein in the present invention, the target protein is cleaved from the fusion protein by the action of an intervening protein such as an intein having an activity of cleaving a peptide bond. More specifically, the method includes steps of isolating a fusion protein, inducing an activity of cleaving a peptide bond of an intervening protein having the activity contained in the isolated fusion protein, and isolating a target protein cleaved from the fusion protein by cleaving a part of the fusion protein by the action of the intervening protein having the activity of cleaving a peptide bond. According to the method of the target protein in the present invention, a target protein that tends to be insoluble or the like alone is expressed as a normal protein by the action of a molecular chaperone, and cleaved without addition of proteases or the like. At this time, the fusion protein may be expressed in a host or in a cell-free translation system.

In the case of expression of the fusion protein in a host to produce a target protein, firstly, a gene of the fusion protein is introduced into the host. For example, an expression vector is constructed by introduction of a gene encoding the fusion protein in an appropriate vector and introduced into an appropriate host to obtain a transformant. Thereupon, the fusion protein is expressed by culture of the transformant and then isolated from the transformant. On the other hand, in the case of expression of the fusion protein in a cell-free translation system, the fusion protein is expressed in a reaction solution that is a cell-free extract of E. coli, a wheat germ, a rabbit reticulocyte, or the like to which nucleotide tri-phosphate or various amino acids added.

Next, the target protein is cleaved from the fusion protein to be isolated by a process of inducing an activity of cleaving a peptide bond of the intervening protein in the isolated fusion protein. In the case that the intervening protein is an intein, the process of inducing the activity of cleaving a peptide bond of the intervening protein includes a pH control, a temperature control, or addition of trigger substances. Specifically, alteration in hydrogen ion concentration so as to be in the range of pH 6 to 8, preferably nearly pH 7 induces the activity of cleaving a peptide bond of an intein. Alteration of temperature so as to be at in the range of 20 to 37°C also induces the activity of an intein. Addition of thiols such as dithiothreitol and β-mercaptoethanol as the trigger substances also induces the activity of an intein. Especially, the thiols are effective in inducing the activity of cleaving an N-intein.

By contraries, it is also effective in increasing an amount of the target protein obtained to prevent the activity of cleaving a peptide bond of an intein from being induced in a host or in a test tube, before the fusion protein being isolated. Adjustment of the temperature for culturing a host to under 20°C or cell-free protein synthesis under 20°C is one of methods therefor.

If and when the intervening protein is a protease, a control of optimal buffer conditions, the temperature, pH, or the like depending on kinds of the protease induces the activity of cleaving a peptide bond.

A host expressing the fusion protein is not particularly limited, but includes bacteria such as E. coli, other prokaryotic cells, yeasts, insect cells, cultured mammalian cells, cultured plant cells, and transgenic animals and plants. In particular, bacteria such as E. coli or yeasts are preferable because of its rapid growth characteristics, easy operations for culturing, and low cost of source of nutrition or the like for culturing. Further, the fusion protein may be expressed either intracellularly or extracellularly in the host, but it is desirable for abundant expression to be intracellularly expressed.

Herein, if and when the host is E. coli, when the size of an expression plasmid is 10 kbp or more, the copy number may be decreased, resulting in a reduction in the amount of the fusion protein synthesized. As a countermeasure, co-expression of the fusion protein in the same host inhibits a reduction in the expression amount. Specifically, the same genes synthesizing the same fusion proteins are introduced into two different vectors such as plasmids having different replication regions and different antibiotic resistant genes, E. coli is transformed with the two kinds of vectors in the presence of the two kinds of antibiotics, and high expression is achieved by synthesizing the fusion protein in the transformant.

Further, according to the method of a target protein in the present invention, a gene encoding the fusion protein and a gene encoding only the molecular chaperone may be respectively introduced into two different vectors such as plasmids each capable of coexistence and replication in the same host and then co-expressed in the same host. If and when the ratio of the number of chaperonin subunits to the number of a target protein is small, a gene encoding a fusion protein and a gene encoding a chaperonin subunit may be respectively introduced into two different plasmids each capable of coexistence and replication in the same host and then co-express the fusion protein and the chaperonin subunit.

It is known that a protein consisting of a partial sequence of an intein and a remaining protein prepared by removal of the protein consisting of a partial sequence of an intein from the full-length of an intein (hereinafter referred to as "a protein consisting of a remaining partial sequence of an intein") have the so-called trans-splicing (Wu H. et al., Biochimica et biophysica acta, 1387, 422-432, 1998). When a protein consisting of a partial sequence of an intein and a protein consisting of a remaining partial sequence of the intein exist adjacent to each other, two adjacent proteins are thought to be easy to be assembled. Herein, to assemble two kinds of proteins means to bring these proteins close to each other and to bind or associate via a covalent bond or a non-covalent bond (hydrogen bond, hydrophobic interaction, intermolecular force, and the like). A protein consisting of a partial sequence of an intein and a protein consisting of a remaining partial sequence of the intein have a function of assembling two kinds of protein (viz. assembly function). The use of the assembly function assembles two kinds of target proteins. Specifically, if and when two kinds of proteins are possible to be precursors of certain target protein, these precursors are assembled to produce the target protein.

Examples in the case that the target protein is an immunoglobulin and the precursors are an antibody light chain and an antibody heavy chain are described below. As for a first example, a fusion protein including the antibody heavy chain linked to the C-terminus of a molecular chaperone via a protein consisting of the C-terminus partial sequence of an intein is produced, whereas a fusion protein including the antibody light chain linked to the N-terminus of another molecular chaperone via a protein consisting of the remaining partial sequence of the intein. Then, these two fusion proteins are brought close to each other and made assembled to produce the immunoglobulin wherein the antibody heavy chain and the antibody light chain are linked via disulfide bonds. As for a second example, a fusion protein including the antibody heavy chain linked to the C-terminus of a molecular chaperon via a protein consisting of the C-terminus partial sequence of an intein and the antibody light chain linked to the N-terminus of the molecular chaperone via a protein consisting of the remaining partial sequence of the intein is produced. Then, the antibody heavy chain and the antibody light chain are assembled by means of the assembly function of the intein to produce the immunoglobulin wherein the antibody heavy chain and the antibody light chain are linked via disulfide bonds. Herein, the protein consisting of the remaining partial sequence of an intein may include a region of endonuclease activity.

### EXAMPLES

Hereinafter, this invention is described in more detail by reference to the Examples, but this invention is not limited to the Examples.

### Example 1

### 1. Isolation of SspI gene

PCR using pTWIN1 (New England Biolabs) as a template and oligonucleotides as shown in SEQ ID No. 1 and No. 2 as a primer set was carried out, thereby isolating a DNA fragment containing a gene encoding SspI. The nucleotide sequence and the amino acid sequence corresponding thereto are shown in SEQ ID No. 3 and only the amino acid sequence is shown in SEQ ID No. 4. Herein, SspI is a modified intein (C-intein) obtained by removal of the endonuclease region of DnaB helicase intein derived from a cyanobacterium, Synechocystis sp. strain PCC 6803 and by substitution of the amino acid of the N-terminal thereof to an alanine.

### 2. Construction of an archaeal chaperonin subunit linkage expression system

Genomic DNA was extracted from Thermococcus strain KS-1 (JCM No. 11816). A DNA fragment containing a chaperonin β subunit (hereinafter referred to as "TCPβ") gene as shown in SEQ ID No. 7 was isolated by PCR using the genomic DNA as a template and oligonucleotides as shown in SEQ ID No. 5 and 6 as a primer set. The DNA fragment containing the amplified TCPβ gene is four times linked to one another in tandem, thereby producing a gene of TCPβ 4-times linkage (hereinafter referred to as "(TCPβ)₄").

Meanwhile, oligonucleotides having complementary nucleotide sequences as shown in SEQ ID No. 36 and No. 37 were synthesized and annealed to give a double-stranded DNA having a multi-cloning site. The double-stranded DNA includes sites of NcoI, BamHI, SspI, and HpaI arranged in order of mention, and a gene encoding a histidine tag (6His, SEQ ID No. 8) consisting of six histidine residues, a termination codon, and an NotI site at the downstream of the sites. Then, the double-stranded DNA was introduced into the NcoI-NotI site of pET21d plasmid (Novagen).

(TCPβ)₄ gene isolated in the present Example was introduced into the BamHI site of the plasmid to construct pETDH(TCPβ)₄. SspI gene isolated in the present Example was introduced into the downstream of the (TCPβ)₄ gene in pETDH(TCPβ)₄ to construct an expression vector pETDH(TCPβ)₄I.

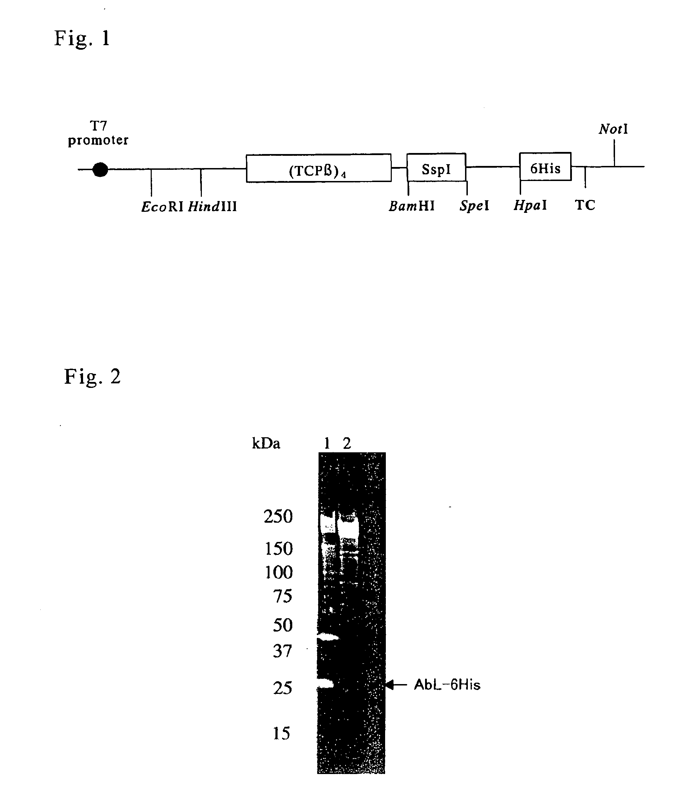

The constitution of pETDH(TCPβ)₄I is shown in Fig. 1. More specifically, pETDH(TCPβ)₄I includes T7 promoter, and (TCPβ)₄ gene, SspI gene, and 6His gene arranged in order of mention at the downstream thereof. Further, a vector expressing a fusion protein composed of (TCPβ)₄, SspI, and a target protein with 6His at its C-terminus was constructed by introduction of a gene encoding the target protein and the termination codon between the SpeI site and the HpaI site of pETDH(TCPβ)₄I.

### 3. Expression and cleavage of an antibody light chain

Long-chain DNA synthesis prepared a DNA fragment containing a light chain (AbL) gene of an anti-human HBs (a surface protein of hepatitis B virus) antibody as shown in SEQ ID No. 9. Herein, the DNA fragment was provided with an SpeI site and an HpaI site respectively at its 5'-terminus and its 3'-terminus both derived from the primers. The DNA fragment was treated with SpeI and HpaI and introduced into the expression vector pETDH(TCPβ)₄I treated in advance with the same restriction enzymes. Thereby, an expression vector pETDH(TCPβ)₄I□AbL synthesizing a fusion protein composed of (TCPβ)₄, SspI, and AbL was constructed.

The obtained expression vector pETDH(TCPβ)₄I□AbL was introduced into E. coli strain BL21 (DE3) (Stratagene) to give a transformant. The transformant was cultured at 30°C in 2xYT medium (Bacto-trypton 16 g, Yeast extract 10 g, and NaCl 5 g/L) containing carbenicillin (100 µg/mL) for 24 hours, whereby a fusion protein composed of (TCPβ)_{4,}, SspI, and AbL was expressed. Then, cells were suspended in 50 mM Tris/HCI buffer (pH 7.0) containing 0.2 mM EDTA to be lysed by sonication in the presence of a protease inhibitor cocktail (Nacalai Tesque), whereupon the supernatant of cell lysate was recovered by centrifugation.

### Comparative Example 1

In order to construct pETDH(TCPβ)₄P, a gene encoding a PreScission protease recognition sequence (SEQ ID No. 10) instead of a gene encoding SspI was introduced between the BamHI site and the SpeI site of pETDH(TCPβ)₄I shown in Fig. 1. An expression vector pETDH(TCPβ)₄P□ AbL was constructed in the same way as Example 1 except for the use of pETDH(TCPβ)₄P instead of pETDH(TCPβ)₄I. Further, as well as Example 1, the supernatant of cell lysate was obtained by transformation and culturing of the transformant.

Whether AbL was cleaved from the chaperonin or not was examined by Western blot analysis using an antibody recognizing an antibody light chain (goat anti-human IgG F(ab')₂ antibody-HRP conjugate, PIERCE) on the supernatants of cell lysates respectively prepared in Example 1 and Comparative Example 1 treated at room temperature for 2 hours. The results are shown in Fig. 2. AbL in the cell lysate in Example 1 was detected adjacent to 25 kDa of a molecular weight, that is, it was confirmed that AbL was cleaved from the fusion protein (lane 1). Herein, a band adjacent to 250 kDa is the fusion protein composed of (TCPβ)₄, SspI, and AbL. On the other hand, AbL in the cell lysate in Comparative Example 1 was detected only adjacent to 250 kDa of a molecular weight, that is, AbL was not cleaved from the fusion protein (lane 2).

### Example 2

### 1. Expression and cleavage of a hepatitis C virus

Long-chain DNA synthesis prepared a DNA fragment containing a hepatitis C virus core antigen (HCc) gene as shown in SEQ ID No. 11. An expression vector pETDH(TCPβ)₄I □ HCc synthesizing a fusion protein composed of (TCPβ)₄, SspI, and HCc was constructed by introduction of the DNA fragment in the expression vector pETDH(TCPβ)₄I in the same way as Example 1. The obtained expression vector pETDH(TCPβ)₄I□HCc was introduced into E. coli strain BL21 (DE3), whereby a transformant was obtained. The transformant was cultured in the same way as Example 1 to give a supernatant of cell lysate.

### Comparative Example 2

A supernatant of cell lysate was obtained by construction of an expression vector, transformation, and culturing of the transformant in the same way as Example 2 except for the use of pETDH(TCPβ)₄P instead of pETDH(TCPβ)₄I.

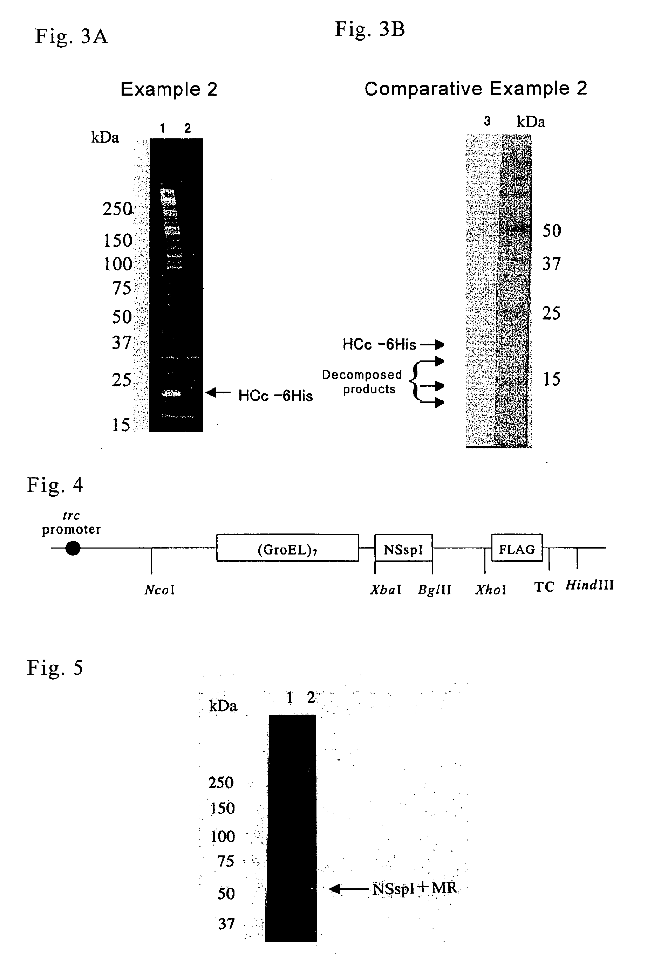

The supernatant of cell lysate in Example 2 was treated at room temperature for 2 hours, whereas the supernatant of cell lysate in Comparative Example 2 was treated with PreScission protease (Amersham Bioscience) at 4°C for 20 hours. Western blot analysis using an antibody recognizing a hepatitis C virus antigen (mouse monoclonal anti-HCV antibody, ABR) was carried out on the respective reaction solutions after the treatments. The results are shown in Fig. 3A (Example 2), and Fig. 3B (Comparative example 2). The lane 1 in Fig. 3A is the sample of Example 2 and the lane 2 is a control of a host alone. The lane 2 in Fig. 3B is the sample of Comparative Example 2.

As shown in Fig. 3A, HCc in the cell lysate in Example 2 was detected adjacent to 21 kDa of a molecular weight, that is, it was confirmed that HCc was cleaved from the fusion protein without being decomposed. On the other hand, as shown in Fig. 3B, signals in the cell lysate were detected not only adjacent to 21 kDa but also adjacent to 18 kDa, 15 kDa, and 12 kDa, that is, it was confirmed that HCc was decomposed. It was thought that HCc had particularly a high hydrophobicity resulting in reduction of stringency of recognition by a restriction protease, with a consequence of internal cleavage of other parts of HCc as well as the recognition sequence.

### Example 3

### 1. Isolation of a gene encoding modified SspI that cleaves only the N-terminus

PCR using pTWIN1 (New England Biolabs) as a template and oligonucleotides as shown in SEQ ID No. 12 and No. 13 as a primer set was carried out, thereby isolating a DNA fragment containing a gene encoding SspI as shown in SEQ ID No. 6 that was modified so as to cleave only the N-terminus (hereinafter referred to as "NSspI"). More specifically, NSspI is a modified intein (N-intein) obtained by removal of the endonuclease region of DnaB helicase intein derived from a cyanobacterium, Synechocystis sp. strain PCC 6803 and by substitution of the amino acid of the C-terminal thereof to an alanine.

### 2. Construction of E. coli chaperonin subunit linkage expression system

Genomic DNA was extracted from E. coli strain HMS174 (Novagen). A DNA fragment containing GroEL subunit gene as shown in SEQ ID No. 17 was isolated by PCR using the genomic DNA as a template and oligonucleotides as shown in SEQ ID No. 15 and 16 as a primer set. Meanwhile, oligonucleotides having complementary nucleotide sequences as shown in SEQ ID No. 38 and No. 39 were synthesized and annealed to give a double-stranded DNA having a multi-cloning site. The double-stranded DNA includes sites of NcoI, XbaI, BgIII, and XhoI arranged in order of mention, and a gene encoding FLAG peptide (SEQ ID No. 18) and a termination codon at the downstream of the sites. Then, the double-stranded DNA was introduced into NcoI-HindIII site of a plasmid pTrc99a (Amersham Bioscience), whereupon the DNA fragment containing GroEL subunit gene amplified above was introduced into the introduced XbaI site. The same operations were repeated to introduce a GroEL subunit 7-times linkage (hereinafter referred to as "(GroEL)₇") gene that contained seven GroEL subunit genes linked to one another in tandem, thereby constructing pT(GroEL)₇. Further, NSspI gene obtained in the present Example was introduced into the XbaI-Bg1II site at the downstream of the (GroEL)₇ gene, whereby an expression vector pT(GroEL)₇NI was constructed. The constitution of pT(GroEL)₇NI is shown in Fig. 4. More specifically, pT(GroEL)₇NI includes trc promoter, and the gene of GroEL subunit 7-times linkage ((GroEL)₇) containing seven GroEL subunit genes arranged in tandem, NSspI gene, and the gene encoding FLAG peptide, all arranged in order of mention at the downstream thereof. Further, a vector expressing a fusion protein composed of (GroEL)₇, NSspI, and a target protein was constructed by introduction of a gene encoding the target protein between the Bg1II site and the XhoI site.

### 3. Expression and cleavage of an acetylcholine muscarinic receptor

Long-chain DNA synthesis prepared a DNA fragment containing an acetylcholine muscarinic receptor (MR) gene as shown in SEQ ID No. 19. The DNA fragment was introduced between the Bg1II site and the XhoI site of the pT(GroEL)₇NI. Thereby, an expression vector pT(GroEL)₇NI□MR synthesizing a fusion protein composed of (GroEL)₇, NSspI, and MR was constructed. In the same way as Example 1 except that the culturing temperature was at 25°C, transformation and culturing of the transformant were carried out by the use of pT(GroEL)₇NI□MR to give a supernatant of cell lysate.

The obtained supernatant was purified with anti-FLAG peptide antibody column and then 40 mM (final concentration) dithiothreitol (DTT) was added to cleave a peptide bond of the N-terminus of NSspI. Western blot analysis using anti-FLAG peptide antibody was carried out. The result is shown in the lane 2 in Fig. 5.

### Comparative Example 3

The obtained supernatant in Example 3 was purified with anti-FLAG peptide antibody column. Without adding DTT, Western blot analysis using anti-FLAG peptide antibody was carried out. The result is shown in the lane 1 in Fig. 5.

In Example 3, a signal of the fusion protein composed of NSspI and MR was detected adjacent to 52 kDa of a molecular weight, that is, it was confirmed that the MR was cleaved from the fusion protein (lane 2). On the other hand, in Comparative Example 3, no signal was detected adjacent to 52 kDa of a molecular weight, that is, it was confirmed that the MR was not cleaved from the fusion protein (lane 1). Thus, a cleaving activity of an intein was induced by DTT.

### Example 4

### 1. Construction of an expression system for a fusion protein with a PPIase

Genomic DNA was extracted from Thermococcus strain KS-1 (JCM No. 11816). A DNA fragment containing PPIase (TPPIase) gene as shown in SEQ ID No. 22 derived from hyperthermophilic archaea was isolated by PCR using the genomic DNA as a template and oligonucleotides as shown in SEQ ID No. 20 and 21 as a primer set.

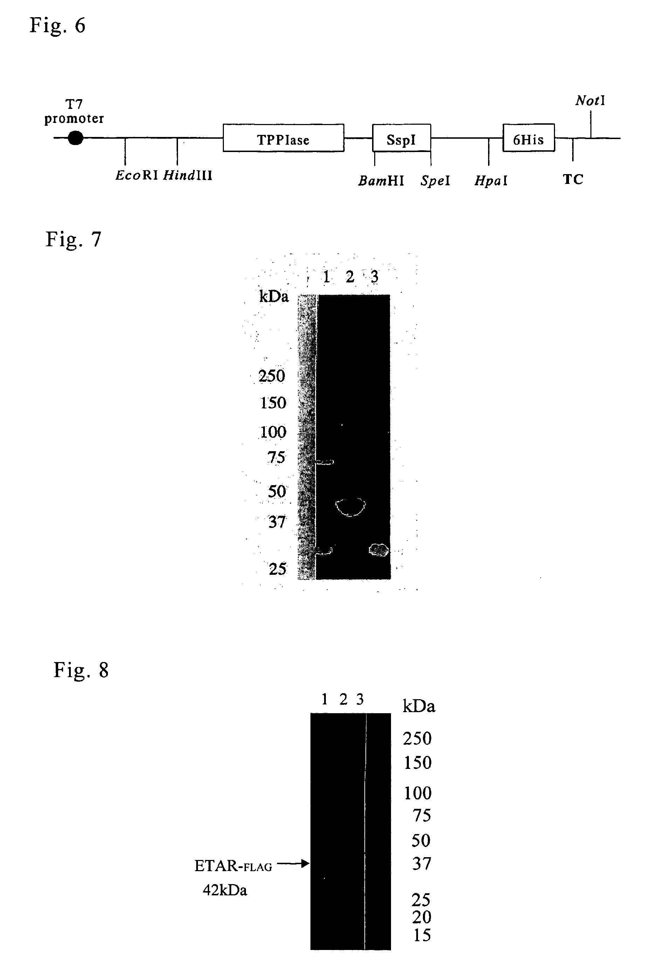

pETDH(TCPβ)₄I prepared in Example 1 was treated with BamHI and NotI, whereby a DNA fragment containing an SspI gene, a His tag gene, and a termination codon was isolated. Meanwhile, oligonucleotide as shown in SEQ ID No. 35 and oligonucleotide having its complementary nucleotide sequence were synthesized and annealed to give a double-stranded DNA fragment. The double-stranded DNA fragment was introduced into an NcoI site of pET21d plasmid (Novagen). Then, a DNA fragment containing the TPPIase gene amplified in the present Example in the NcoI-SpeI site contained in the introduced DNA fragment. A DNA fragment between the BamHI site contained in the introduced oligonucleotide and the NotI site in the multi-cloning site of the pET21d was excised, and instead, the DNA fragment containing the SspI gene, the His gene, and the termination codon isolated above was introduced, whereby pET TPPIase I was constructed. The constitution of pET TPPIase I is shown in Fig. 6. More specifically, pET TPPIase I includes T7 promoter and TPPIase gene, SspI gene, and 6His gene arranged in order of mention at the downstream thereof. Further, a vector expressing a fusion protein composed of TPPIase, SspI, and a target protein was constructed by introduction of a gene encoding the target protein between the SpeI site and the HpaI site.

### 2. Expression and cleavage of green fluorescein protein (GFP)

PCR using pIVEX2.3-GFP WT (Roche Diagnostics) as a template and oligonucleotides as shown in SEQ ID No. 23 and No. 24 as a primer set was carried out, thereby isolating a DNA fragment containing a gene of green fluorescein protein (GFP) as shown in SEQ ID No. 25. Herein, the DNA fragment was provided with an SpeI site and an HpaI site respectively at its 5'-terminus at its 3'-terminus both derived from the primers. The DNA fragment was treated with SpeI and HpaI and introduced into the expression vector pET TPPIase I treated in advance with the same restriction enzymes. Thereby, an expression vector pET TPPIase I□GFP synthesizing a fusion protein composed of a hyperthermophilic archaeal PPIase (TPPIase), SspI, and GFP was constructed.

The obtained expression vector pET TPPIase I□GFP was introduced into E. coli strain BL21 (DE3) to give a transformant. The transformant was cultured at 25°C in 2xYT medium containing carbenicillin (100 µg/mL) for 24 hours, whereby a fusion protein composed of TPPIase, SspI, and GFP was expressed. Then, cells were suspended in 50 mM Tris/HCI buffer (pH 7.0) to be lysed by sonication in the presence of a protease inhibitor cocktail, whereupon the supernatant of cell lysate was obtained by centrifugation.

### Comparative Example 4

In the process of constructing pET TPPIase I as shown in Fig. 1, a gene encoding a PreScission protease recognition sequence instead of the gene encoding SspI was introduced between the BamHI site and the SpeI site, whereby pET TPPIase P was constructed.

A supernatant of cell lysate was recovered by construction of an expression vector, transformation, and culturing of the transformant in the same way as Example 4 except for the use of pET TPPIase P instead of pET TPPIase I.

Whether GFP was cleaved from TPPIase or not was examined by Western blot analysis using an antibody recognizing a histidine tag (mouse monoclonal anti-His antibody, Amersham Biosciences) on the supernatants of cell lysates respectively prepared in Example 4 and Comparative Example 4, both treated at room temperature for 2 hours after purification with Ni-NTA agarose gel (Qiagen). The results are shown in Fig. 7. The GFP in the cell lysate in Example 4 (lane 1) was detected adjacent to 27 kDa of a molecular weight, that is, it was confirmed that the GFP was cleaved from the fusion protein (lane 1). Herein, a signal adjacent to 62 kDa in the lane 1 is the fusion protein composed of TPPIase, SspI, and GFP. On the other hand, a signal in the cell lysate in Comparative Example 4 (lane 2) was detected only adjacent to 45 kDa of a molecular weight of the fusion protein composed of TPPIase and GFP, that is, the GFP was not cleaved therefrom. The lane 3 is GFP standard.

### Example 5

### 1. Expression and cleavage of an endothelin receptor

pT(GroEL)₇I was constructed in the same way as Example 3 by use of the SspI gene prepared in Example 1. pT(GroEL)₇I is a vector having the SspI gene introduced instead of NSspI gene of pT(GroEL)₇NI as shown in Fig. 4. Meanwhile, PCR using a human cDNA library (Takara Bio) as a template and oligonucleotides as shown in SEQ ID No. 26 and No. 27 as a primer set was carried out, thereby isolating a DNA fragment containing an endothelin A receptor (ETAR) gene as shown in SEQ ID No. 28. The DNA fragment was introduced between the Bg1II and the XhoI sites of pT(GroEL)₇I. Thereby, an expression vector pT(GroEL)₇I □ ETAR synthesizing a fusion protein composed of (GroEL)₇, SspI, and ETAR was constructed. A transformant was produced by use of the expression vector in the same way as Example 1. The transformant was cultured at 18°C and at 110 rpm to give cells, from which a supernatant of cell lysate was obtained in the same way as Example 1. After the obtained supernatant was purified with anti-FLAG peptide antibody column, Western blot analysis using anti-FLAG peptide antibody (anti-FLAG M2 monoclonal antibody, SIGMA) was carried out to examine whether a peptide bond of the C-terminus of the SspI was cleaved or not. The result is shown in the lane 2 in Fig. 8.

### Comparative Example 5

The obtained supernatant in Example 5 was cultured in the same way as Example 5 except that the culturing temperature was at 25°C not at 18°C to give a supernatant of cell lysate. After the supernatant was purified with anti-FLAG peptide antibody column, Western blot analysis using anti-FLAG peptide antibody was carried out to examine whether a peptide bond of the C-terminus of SspI was cleaved or not. The result is shown in the lane 3 in Fig. 8.

In the cell lysate in Example 5, a signal of ETAR was detected adjacent to 42 kDa of a molecular weight, that is, it was confirmed that ETAR was cleaved from the fusion protein (lane 2). On the other hand, in the cell lysate in Comparative Example 5, no band was detected adjacent to 42 kDa of a molecular weight, that is, it was confirmed that ETAR was not cleaved from the fusion protein (lane 3). Herein, the lane 1 is a control using a vector without SspI gene.

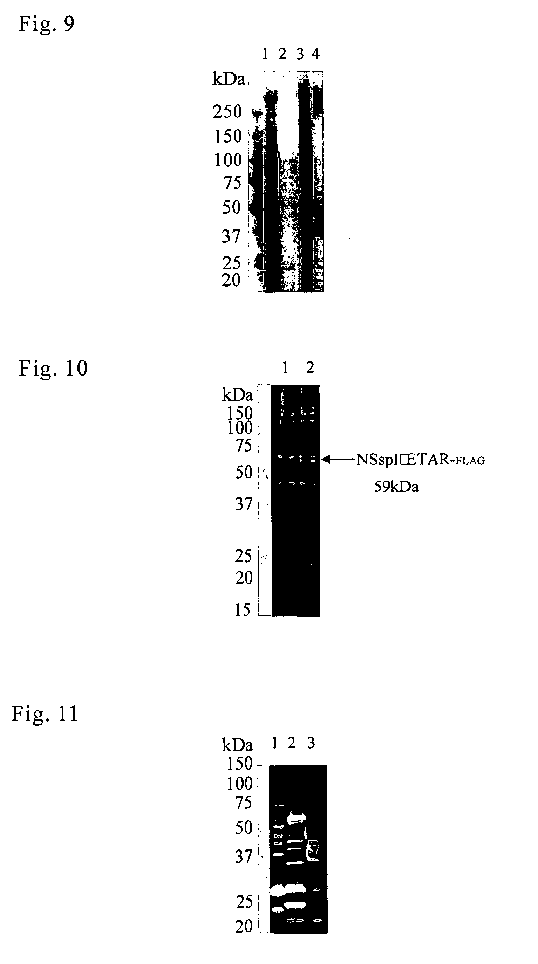

Further, SDS-PAGE analysis with CBB straining on a fraction in which the supernatant of cell lysate obtained in Comparative Example 5 was purified with anti-FLAG peptide antibody column was carried out to examine presence or absence of a band. As a control, SDS-PAGE analysis was also carried out on a fraction, which is cultured, lysed, and purified as well, of a fusion protein composed of (GroEL)₇ and ETAR without SspI. The results are shown in Fig. 9. In Fig. 9, the lane 1 shows the supernatant of cell lysate obtained in Comparative Example 5, the lane 2 shows a fraction of the supernatant purified with FLAG column, the lane 3 shows the supernatant of cell lysate of a control, and the lane 4 shows a fraction of the supernatant of the control purified with FLAG column. As a result, in the case of the fusion protein composed of (GroEL)₇ and ETAR without SspI, a band was detected in the fraction purified with an anti-FLAG peptide antibody column (lane 4). On the other hand, in the case of the fusion protein composed of (GroEL)₇ and ETAR with SspI, no band was detected in the fraction purified with an anti-FLAG peptide antibody column (lane 2). More specifically, a protein binding to anti-FLAG peptide antibody column was not detected in the sample in the lane 2. It means that the band detected in the lane 1 is a protein composed of only (GroEL)₇ without FLAG peptide. That suggests that ETAR with FLAG peptide was cleaved and further decomposed by the action of SspI by culturing at 25°C.

### Example 6

### 1. Expression and cleavage of an endothelin receptor (2)

A DNA fragment containing an endothelin A receptor (ETAR) gene as shown in SEQ ID No. 28 was isolated as well as Example 5.
The DNA fragment was introduced between the Bg1II and the XhoI sites of pT(GroEL)₇NI prepared in Example 3. Thereby, an expression vector pT(GroEL)₇NI□ETAR synthesizing a fusion protein composed of (GroEL)₇, NSspI, and ETAR was constructed. By use of pT(GroEL)₇NI□ETAR, a supernatant of cell lysate was obtained by transformation and culturing of the transformant in the same way as Example 1. After the obtained supernatant was purified with anti-FLAG peptide antibody column, DTT was added so as to get a final concentration of 40 mM to cleave a peptide bond of the N-terminus of the NSspI. The result of Western blot analysis using anti-FLAG peptide antibody is shown in the lane 2 in Fig. 10.

### Comparative Example 6

The obtained supernatant of cell lysate in Example 6 was purified with anti-FLAG peptide antibody column, whereupon Western blot analysis using anti-FLAG peptide antibody was carried out without adding DTT, and its result is shown in the lane 1 in Fig. 10.

In the cell lysate in Example 5, a signal of the fusion protein composed of NSspI and ETAR was detected adjacent to 59 kDa of a molecular weight, that is, it was confirmed that ETAR was cleaved from the fusion protein (lane 2). On the other hand, in the cell lysate in Comparative Example 5, no signal was detected adjacent to 59 kDa of a molecular weight, that is, it was confirmed that ETAR was not cleaved from the fusion protein (lane 1). In this way, a cleaving activity of an intein was induced by DTT.

### Example 7

### 1. Expression and cleavage of GFP (2); Cell-free protein synthesis

A protein was synthesized in the same way as Example 4 except that a cell-free protein expression (using RTS500, Roche Diagnostics) was carried out instead of culturing of E. coli.

### Comparative Example 7

A protein was synthesized in the same way as Comparative Example 4 except that a cell-free protein expression (using RTS500, Roche Diagnostics) was carried out instead of culturing of E. coli.

Whether GFP was cleaved from TPPIase or not was examined by Western blot analysis using anti-GFP mouse monoclonal antibody recognizing a histidine tag (Santa Cruz Biotechnology) on the reaction solutions after protein syntheses respectively prepared in Example 7 and Comparative Example 7, which were treated at room temperature for 2 hours after purification with Ni-NTA agarose gel (Qiagen). The results are shown in Fig. 11. The lane 1 is a control using GFP-6His. GFP in pET TPPIase I□GFP in Example 7 (lane 2) was detected adjacent to 27 kDa of a molecular weight, that is, it was confirmed that GFP was cleaved from TPPIase. Herein, a signal adjacent to 62 kDa is the fusion protein composed of TPPIase, SspI, and GFP. On the other hand, a signal in Comparative Example 7 (lane 3) was detected only adjacent to 45 kDa of a molecular weight of the fusion protein composed of TPPIase and GFP, that is, GFP was not cleaved therefrom.

### Example 8

### 1. Isolation of MMI gene

PCR using pTWIN1 (New England Biolabs) as a template and oligonucleotides as shown in SEQ ID No. 29 and No. 30 as a primer set was carried out, thereby isolating a DNA fragment containing a gene encoding MMI (MMI gene), which is the C-terminus partial sequence of SspI. The nucleotide sequence and an amino aced sequence corresponding thereto are shown in SEQ ID No. 31 and only the amino acid sequence is shown in SEQ ID No. 32. More specifically, the MMI consists of the amino acids from the C-terminus to 48^{th} of the SspI.

### 2. Construction of an archaeal chaperonin subunit linkage expression system

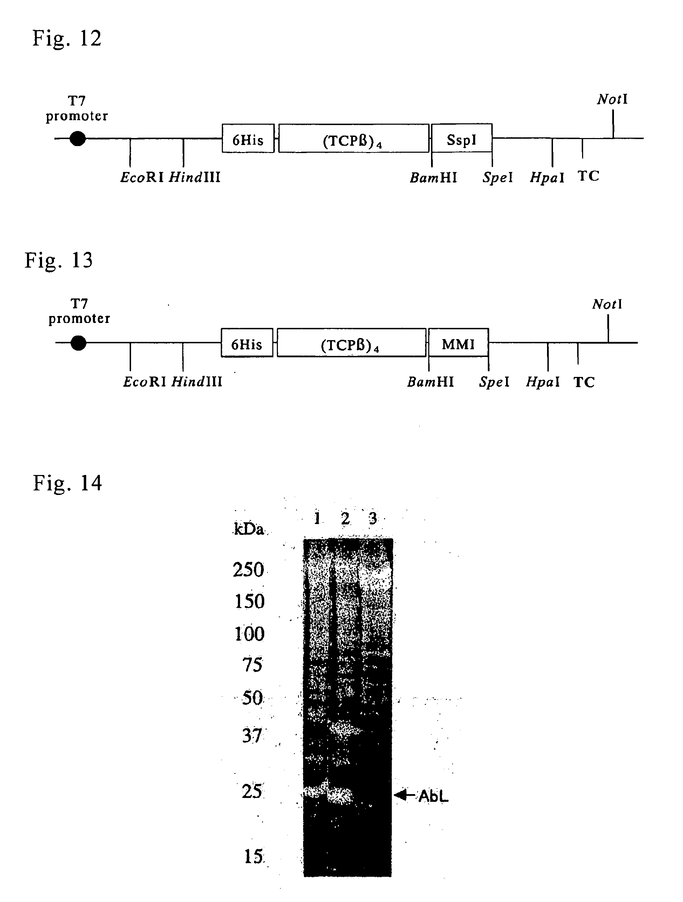

Two kinds of oligonucleotides having complementary nucleotide sequences as shown in SEQ ID No. 40 and No. 41 were synthesized and annealed to give a double-stranded DNA encoding 6His. The double-stranded DNA was introduced into the NcoI site at the upstream of (TCPβ)₄ gene on the expression vector pETDH(TCPβ)₄I prepared in Example 1, whereby an expression vector pETDH(TCPβ)₄I-2 was constructed. The constitution of pETDH(TCPβ)₄I-2 is shown in Fig. 12. More specifically, pETDH(TCPβ)₄I-2 includes T7 promoter and 6His gene, (TCPβ)₄ gene, and SspI gene arranged in order of mention at the downstream thereof. Further, a vector expressing a fusion protein composed of (TCPβ)₄, SspI, and a target protein with His tag at its N-terminus was constructed by introduction of a gene encoding the target protein between the SpeI site and the HpaI site of pETDH(TCPβ)₄I-2.

As well, an expression vector pETDH(TCPβ)₄MMI-2 into which a MMI gene was introduced instead of SspI of pETDH(TCPβ)₄I-2 was constructed. The constitution of pETDH(TCPβ)₄MMI-2 is shown in Fig. 13. More specifically, the constitution of pETDH(TCPβ)₄MMI-2 is substantially the same as that of pETDH(TCPβ)₄I-2 except for the use of the MMI gene instead of the SspI gene. A vector expressing a fusion protein composed of (TCPβ)₄, MMII, and a target protein with His tag at its N-terminus was constructed by introduction of a gene encoding the target protein between the SpeI site and the HpaI site of pETDH(TCPβ)₄MMI-2.

### 3. Expression and cleavage of an antibody light chain

The DNA fragments each containing AbL gene isolated in Example 1 were respectively introduced into pETDH(TCPβ)₄I-2 and pETDH(TCPβ)₄MMI-2, whereby pETDH(TCPβ)₄I □ AbL-2 and pETDH(TCPβ)₄MMI□AbL-2 were constructed. More specifically, a fusion protein composed of (TCPβ)₄, SspI, and AbL is expressed by use of pETDH(TCPβ)₄I□AbL-2, and pETDH(TCPβ)₄MMI□AbL-2 is expressed by use of a fusion protein composed of (TCPβ)₄, MMI, and AbL.

The obtained expression vectors pETDH(TCPβ)₄I □ AbL-2 and pETDH(TCPβ)₄MMI□AbL-2 were respectively introduced into E. coli strain BL21 (DE3) to give transformants. These transformants were cultured at 30°C in 2xYT medium containing carbenicillin (100 µg/mL) for 24 hours, whereby a fusion protein composed of (TCPβ)₄, SspI, and AbL and a fusion protein composed of (TCPβ)₄, MMI, and AbL were expressed. Each of these cells was suspended in 50 mM Tris/HCI buffer (pH 7.0) containing 0.2 mM EDTA to be lysed by sonication in the presence of the protease inhibitor cocktail, whereupon each of supernatants of cell lysates was recovered by centrifugation.

### Comparative Example 8

An expression vector pET(TCPβ)₄P □ AbL-2 was constructed by introduction of a gene encoding a PreScission protease recognition amino acid sequence instead of SspI gene of pETDH(TCPβ)₄I□AbL-2 constructed in Example 8.

Whether AbL was cleaved from the chaperonin or not was examined by Western blot analysis using an antibody recognizing an antibody light chain on the supernatants of cell lysates respectively prepared in Example 8 and Comparative Example 8, which were treated at room temperature for 2 hours. The results are shown in Fig. 14. More specifically, AbLs in pETDH(TCPβ)₄MMI□AbL-2 (lane 1) and pETDH(TCPβ)₄I□ AbL-2 (lane 2) in Example 8 were detected adjacent to 25 kDa of a molecular weight, that is, it was confirmed that each AbL was cleaved from each of the fusion proteins. Herein, a band adjacent to 250 kDa is the fusion protein composed of (TCPβ)₄, MMI, and AbL (lane 1) or the fusion protein composed of (TCPβ)₄, SspI, and AbL (lane 2). On the other hand, AbL in Comparative Example 8 was detected only adjacent to 250 kDa of a molecular weight, that is, AbL was not cleaved from the fusion protein (lane 3). Comparing strength of the signal of the lane 1 to that of the lane 2, they were equal to each other. More specifically, MMI and SspI had substantially the same activity of cleaving a target protein.

### Example 9

### 1. Expression of an antibody heavy chain

Long-chain DNA synthesis prepared a DNA fragment containing a heavy chain (AbH) gene of an anti-human HBs antibody heavy chain as shown in SEQ ID No. 33. Herein, the DNA fragment was provided with an SpeI site and an HpaI site respectively at its 5'-terminus and at its 3'-terminus both derived from the primers. The DNA fragment was introduced between the SpeI site and the HpaI site of pETDH(TCPβ)₄MMI-2 as shown in Example 8. Thereby, an expression vector pETDH(TCPβ)₄MMI□AbH-2 synthesizing a fusion protein composed of (TCPβ)₄, MMI, and AbH with 6His at its N-terminus was constructed.

The obtained expression vector pETDH(TCPβ)₄MMI □ AbH-2 was introduced into E. coli strain BL21 (DE3) to give a transformant. The transformant was cultured at 30°C in 2xYT medium containing carbenicillin (100 µg/mL) for 24 hours, whereby a fusion protein composed of (TCPβ)_{4,}, MMI, and AbH was expressed. Cells were harvested and suspended in 50 mM Tris/HCI buffer (pH 7.0) containing 0.2 mM EDTA to be lysed by sonication in the presence of the protease inhibitor cocktail, whereupon a supernatant of cell lysate was recovered by centrifugation.

### Comparative Example 9

In the same way as Example 9 except for the use of pETDH(TCPβ)₄I-2 instead of pETDH(TCPβ)₄MMI-2, a supernatant of cell lysate was recovered by construction of an expression vector, transformation, and culturing of the transformant.

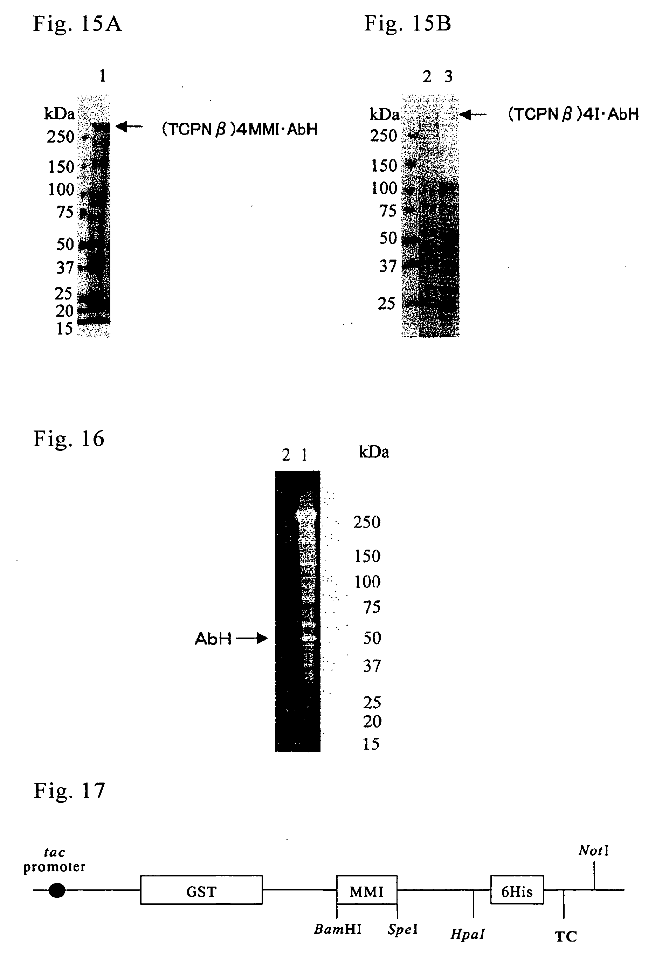

Each of the supernatants of cell lysates obtained in Example 9 and Comparative Example 9 was subjected to SDS-PAGE analysis with CBB straining. The results are shown in Fig. 15. Specifically, in the lane 1 (Example 9), a band derived from the fusion protein composed of (TCPβ)_{4,}, MMI, and AbH was detected adjacent to 290 kDa. In the lane 2 (Comparative Example 9), only an extremely weak band was detected adjacent to 300 kDa. More specifically, the expression amount in Comparative Example 9 was extremely lower than that in Example 9.

As described above, in the case of the fusion protein composed of (TCPβ)₄, MMI, and AbH wherein the target protein is AbH of about 50 kDa, it was thought that SspI and AbH were not accommodated in the cavity of the chaperonin, resulting in no expression or radical reduction of the expression amount. On the other hand, as Example 9, when MMI, which was a partial sequence of the SspI, instead of SspI, was introduced, the fusion protein was expressed without problems. This was attributed to the fact that the MMI and AbH were accommodated in the cavity of the chaperonin.

AbH was cleaved from the fusion protein by treatment of the supernatant of cell lysate of the expression vector pETDH(TCPβ)₄MMI□ AbH obtained in Example 9 at room temperature for 2 hours. Whether AbH was cleaved from the fusion protein or not was examined by Western blot analysis using an antibody recognizing an antibody heavy chain (goat anti-human IgG F(ab')₂ antibody-HRP conjugate, PIERCE). The results are shown in Fig. 16. As a result, a signal was detected adjacent to 50 kDa of a molecular weight, that is, it was confirmed that AbH was cleaved from the fusion protein. Herein, a band adjacent to 290 kDa is a fusion protein composed of a chaperonin β subunit TCPβ tetramer, MMI, and AbH.

### Example 10

### 1. Construction of an expression system for a fusion protein with glutathione-S-transferase (GST)

A double-stranded DNA as shown SEQ ID No. 34 was introduced into BamHI-NotI site of a vector pGEX-4T-1 commercially available from Amersham Pharmacia Biotech, and then an SpeI site, an HpaI site and 6His gene were introduced into the vector. Further the MMI gene obtained in Example 8 was introduced between the BamHI site and the SpeI site. The resulting expression vector was designated as pGEX MMI. The constitution of the pGEX MMI is shown in Fig. 17. Specifically, the pGEX MMI includes tac promoter and GST gene, MMI gene, and 6His gene arranged in order of mention at the downstream thereof. Further, a vector expressing a fusion protein composed of GST, MMI, and a target protein was constructed by introduction of a gene encoding the target protein between the SpeI site and the HpaI site.

### 2. Expression and cleavage of GFP

PCR using a vector pIVEX2.3-GFP WT (Roche Diagnostics) as a template and oligonucleotides as shown in SEQ ID No. 23 and No. 24 as a primer set was carried out, thereby isolating a DNA fragment containing GFP gene as shown in SEQ ID No. 25. Herein, the DNA fragment was provided with an SpeI site and an HpaI site respectively at its 5'-terminus and at its 3'-terminus both derived from the primers. The DNA fragment was introduced between the SpeI and the HpaI sites in the expression vector pGEX MMI constructed in the present Example. Thereby, an expression vector pGEX MMI□GFP synthesizing a fusion protein composed of GST, MMI, and GFP was constructed. As well, an expression vector pGEX I□ GFP was constructed by introduction of the SspI gene prepared in Example 1 instead of the MMI gene.

The pGEX MMI □ GFP (Example 10-1) and the pGEX I □ GFP (Example 10-1) were respectively introduced into E. coli strain BL21 (DE3) to give transformants. These transformants were cultured at 25°C in 2xYT medium containing carbenicillin (100 µg/mL) for 24 hours, whereby a fusion protein composed of GST, SspI, and GFP and a fusion protein composed of GST, MMI, and GFP were expressed. Each of cells was harvested and suspended in 50 mM Tris/HCI buffer (pH 7.0) to be lysed by sonication in the presence of the protease inhibitor cocktail, whereupon each of the supernatants of cell lysates was recovered by centrifugation.

### Comparative Example 10

An expression vector pGEX P was constructed by introduction of a gene encoding a PreScission protease recognition sequence instead of the gene encoding the MMI between the BamHI site and the SpeI site of the pGEX MMI as shown in Fig. 17. Then, a supernatant of cell lysate was recovered by construction of an expression vector, transformation, and culturing of the transformant in the same way as Example 10 except for the use of the pGEX P instead of the pGEX MMI.

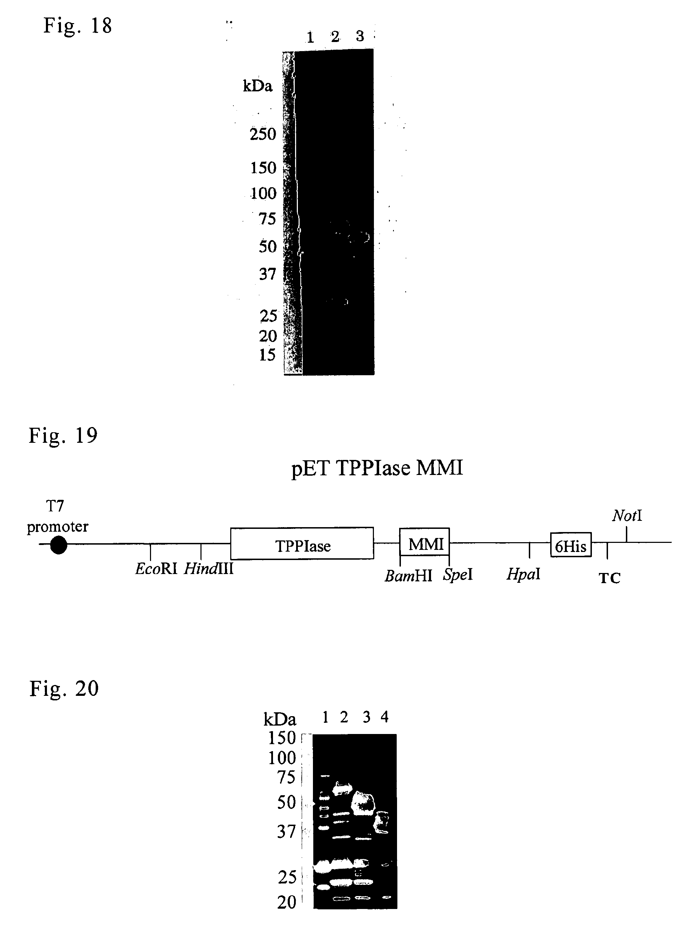

Whether each GFP was cleaved from each of the fusion proteins or not was examined by Western blot analysis using an antibody recognizing a histidine tag on the supernatants of cell lysates respectively prepared in Example 10 and Comparative Example 10, which were treated at room temperature for 2 hours after purification with Ni-NTA agarose gel (Qiagen). The results are shown in Fig. 18. GFP in pGEX I□GFP in Example 10 (lane 1) was detected adjacent to 27 kDa of a molecular weight, that is, it was confirmed that GFP was cleaved from the fusion protein. GFP in pGEX MMI□GFP (lane 2) was also detected adjacent to 27 kDa of a molecular weight, that is, it was confirmed that GFP was cleaved from the fusion protein. Herein, a band adjacent to 48 kDa is the fusion protein composed of GST, MMI, and GFP. On the other hand, in Comparative Example 10 (lane 3), a band was detected only adjacent to 53 kDa of a molecular weight, that is, GFP was not cleaved therefrom.

### Example 11

### 1. Expression of a fusion protein in a cell-free translation system

A plasmid pET TPPIase MMI in which MMI gene was introduced instead of SspI gene in the process of constructing pET TPPIase I prepared in Example 4 was constructed (Fig. 19). A DNA fragment containing GFP gene prepared in Example 10 as shown in SEQ ID No. 25 was treated with SpeI and HpaI and introduced into the expression vector pET TPPIase MMI treated in advance with the restriction enzymes. Thereby, an expression vector pET TPPIase MMI□GFP synthesizing a fusion protein composed of TPPIase, MMI and GFP was constructed.

As to the obtained expression vector pET TPPIase MMI□GFP, a cell-free protein expression (using RTS500, Roche Diagnostics) was carried out.

### Comparative Example 11

pET TPPIase I constructed in Example 4 was used (Comparative Example 11-1). Further, pET TPPIase P was constructed by introduction of a gene encoding a PreScission protease recognition sequence, not MMI gene, between the BamHI site and the SpeI site of the pET TPPIase MMI (Comparative Example 11-2). A protein was synthesized in the same way as Example 11 except for the use of the pET TPPIase I□GFP or the pET TPPIase P□GFP instead of the pET TPPIase MMI□GFP.

Whether each GFP was cleaved from each TPPIase or not was examined by Western blot analysis using anti-GFP mouse monoclonal antibody (Santa Cruz Biotechnology) on the reaction solutions each prepared in Example 11 and Comparative Example 11, which were treated at room temperature for 2 hours after purification with Ni-NTA agarose gel (Qiagen). The results are shown in Fig. 20. The GFP in pET TPPIase MMI□GFP in Example 11 (lane 3) was detected adjacent to 27 kDa of a molecular weight, that is, it was confirmed that GFP was cleaved from TPPIase. Herein, a signal adjacent to 50 kDa is the fusion protein composed of TPPIase, SspI, and GFP. GFP in pET TPPIase I□GFP in Comparative Example 11-1 (lane 2) was also detected adjacent to 27 kDa of a molecular weight, that is, it was confirmed that GFP was cleaved from TPPIase. Herein, a signal adjacent to 62 kDa is the fusion protein composed of TPPIase, SspI, and GFP. On the other hand, a signal in the pET TPPIase P□GFP in Comparative Example 11-2 (lane 4) was detected only adjacent to 45 kDa of a molecular weight of the fusion protein composed of TPPIase and GFP, that is, GFP was not cleaved therefrom. According to these results, it was confirmed that the MMI, as well as the SspI, had an activity of cleaving even in the case of a cell-free protein synthesis.

## Claims

1. A fusion protein, comprising:
an intervening protein having an activity of cleaving a peptide bond;
a molecular chaperone; and
a target protein,
wherein the intervening protein has one terminus to which one selected from a group consisting of the molecular chaperone and its subunit is linked with a peptide bond; and
wherein the intervening protein has the other terminus to which the target protein is linked with a peptide bond.

2. The fusion protein as defined in claim 1,
wherein the molecular chaperone is a chaperonin composed of a plurality of chaperonin subunits.

3. The fusion protein as defined in claim 2,
wherein the intervening protein having an activity of cleaving a peptide bond is linked to one selected from a group consisting of an independent chaperonin subunit and a chaperonin subunit linkage,
the chaperonin subunit linkage being composed of 2 to 10 chaperonin subunits serially linked to one another with a peptide bond.

4. The fusion protein as defined in claim 3,
wherein the intervening protein having an activity of cleaving a peptide bond is linked to at least one site selected from a group consisting of the N-terminus of either the independent chaperonin subunit or the chaperonin subunit linkage, the C-terminus of either the independent chaperonin subunit or the chaperonin subunit linkage, and a linking site of the chaperonin subunits of the chaperonin subunit linkage.

5. The fusion protein as defined in claim 3 or 4,
wherein the ratio of the number of the chaperonin subunits to the number of the target protein is in the range of from 1 : 1 to 10 : 1.

6. The fusion protein as defined in claim 1,
wherein the molecular chaperone is a peptidyl prolyl cis-trans isomerase.

7. The fusion protein as defined in claim 6,
wherein the intervening protein having an activity of cleaving a peptide bond is linked to the N-terminus and/or the C-terminus of the peptidyl prolyl cis-trans isomerase.

8. The fusion protein as defined in one of claims 1 to 7,
wherein the molecular chaperone is derived from one selected from a group consisting of bacteria, archaea, and eukaryotes.

9. The fusion protein as defined in one of claims 1 to 8,
wherein the intervening protein having an activity of cleaving a peptide bond is one selected from a group consisting of an intein and a protein consisting of a partial sequence of an intein.

10. The fusion protein as defined in claim 9,
the intein cleaving only the N-terminus, but not the C-terminus.

11. The fusion protein as defined in claim 9,
the intein cleaving only the C-terminus, but not the N-terminus.

12. The fusion protein as defined in claim 9,
the protein consisting of a partial sequence of an intein including 20 to 120 amino acid residues from the C-terminus of the intein.

13. The fusion protein as defined in one of claims 9 to 12,
the intein being derived from Synechocystis sp.

14. The fusion protein as defined in one of claims 9 to 12,
the intein being derived from one selected from a group consisting of Mycobacterium xenopi, Saccharomyces cerevisiae, and Halobacterium sp.

15. The fusion protein as defined in claim 12,
the protein consisting of a partial sequence of an intein being selected one from a group consisting of a protein having an amino acid sequence as shown in SEQ ID No. 1, and a protein having an amino acid sequence wherein at least one amino acid is deleted, substituted, added, or inserted and having an activity of cleaving a peptide bond.

16. The fusion protein as defined in claim 12,
the protein consisting of a partial sequence of an intein having homology of at least 50% or more to an amino acid sequence as shown in SEQ ID No. 1 and having an activity of cleaving a peptide bond.

17. The fusion protein as defined in one of claims 1 to 16,
wherein the intervening protein having an activity of cleaving a peptide bond is a protease.

18. An isolated gene encoding the fusion protein as defined in one of claims 1 to 17.

19. A method of producing a target protein, the method comprising a step of cleaving the target protein from the fusion protein as defined in one of claims 1 to 17 by the action of the intervening protein having an activity of cleaving a peptide bond.

20. A method of producing a target protein, the method comprising steps of:
preparing the fusion protein as defmed in one of claims 1 to 17;
inducing an activity of cleaving a peptide bond of the intervening protein having the activity contained in the fusion protein prepared in the precedent step; and
isolating the target protein cleaved from the fusion protein by cleaving a part of the fusion protein by the action of the intervening protein having the activity of cleaving a peptide bond induced in the precedent step.

21. The method of producing the target protein as defined in claim 20,
wherein the activity of cleaving a peptide bond is induced by exposure of the fusion protein at a temperature of 20 to 37°C.

22. The method as defined in claim 20 or 21,
wherein the activity of cleaving a peptide bond is induced by exposure of the fusion protein at a hydrogen ion concentration of pH 6 to 8.

23. The method as defined in one of claims 20 to 22,
wherein the activity of cleaving a peptide bond is induced by addition of a thiol.

24. The method as defined in claim 19, the method comprising steps of:
preparing an expression vector having a gene encoding the fusion protein introduced;
introducing the expression vector obtained in the precedent step into a host so as to express the fusion protein; and
cleaving a target protein from the fusion protein obtained in the precedent step.

25. The method as defined in claim 19, the method comprising steps of:
introducing genes encoding the fusion protein into two different plasmids capable of coexistence and replication in the same host so as to produce two kinds of expression vectors;
introducing the two kinds of expression vectors obtained in the precedent step into the same host so as to express the fusion protein; and
cleaving a target protein from the fusion protein obtained in the precedent step by the action of the intervening protein having an activity of cleaving a peptide bond.

26. The method as defined in claim 19, the method comprising steps of:
introducing a gene encoding the fusion protein into one of two different plasmids capable of coexistence and replication in the same host and a gene encoding only a molecular chaperone into the other of the plasmids so as to produce two kinds of expression vectors;
introducing the two kinds of expression vectors obtained in the precedent step into the same host so as to express the fusion protein and the molecular chaperone; and
cleaving a target protein from the fusion protein obtained in the precedent step by the action of the intervening protein having an activity of cleaving a peptide bond.

27. The method as defined in one of claims 24 to 26,
the host being one selected from a group consisting of bacteria, yeasts, animal cells, plant cells, insect cells, animals, plants, and insects.

28. The method as defined in one of claims 19 to 26,
wherein the fusion protein is expressed in the host,
the host being one selected from a group consisting of bacteria, yeasts, animal cells, plant cells, insect cells, animals, plants, and insects.

29. The method as defined in claim 19,
being performed in a cell-free translation system.

30. The method as defined in one of claims 19 to 29,
wherein the molecular chaperone is a chaperonin forming a chaperonin ring constituted by five to ten chaperonin subunits assembled in a ring, and
wherein the target protein is accommodated in the chaperonin ring.

31. A protein consisting of a partial sequence of an intein composed of 20 to 120 amino acid residues from the C-terminus of the intein.

32. The protein consisting of a partial sequence of an intein as defined in claim 31,
wherein the intein is derived from Synechocystis sp.

33. The protein consisting of a partial sequence of an intein as defined in claim 31,
wherein the intein is derived from one selected from a group consisting of Mycobacterium xenopi, Saccharomyces cerevisiae, and Halobacterium sp.

34. The protein consisting of a partial sequence of an intein as defined in claim 31,
being one selected from a group consisting of a protein having an amino acid sequence as shown in SEQ ID No. 1, and a protein having an amino acid sequence wherein at least one amino acid is deleted, substituted, added, or inserted and having an activity of cleaving a peptide bond.

35. The protein consisting of a partial sequence of an intein as defmed in claim 31,
having homology of at least 50% or more to an amino acid sequence as shown in SEQ ID No. 1, and
having an activity of cleaving a peptide bond.

36. An isolated gene encoding the protein consisting of a partial sequence of an intein as defined in one of claims 31 to 35.

37. An expression vector containing the gene as defined in claim 18 or 36.

38. A transformant containing the expression vector as defined in claim 37.

39. A method of producing a target protein, the method comprising steps of:
a first step of producing a first fusion protein composed of a protein consisting of a partial sequence of an intein, one selected from a group consisting of a molecular chaperone and its subunit linked to one terminus of the protein consisting of a partial sequence of an intein with a peptide bond, and a first precursor of target protein linked to the other terminus of the protein consisting of a partial sequence of an intein with a peptide bond;
a second step of producing a second fusion protein composed of a protein consisting of a remaining partial sequence of the intein, one selected form a group consisting of a molecular chaperone and its subunit linked to one terminus of the protein consisting of a remaining partial sequence of an intein with a peptide bond, and a second precursor of target protein linked to the other terminus of the protein consisting of a remaining partial sequence of an intein with a peptide bond;
a third step of cleaving the first precursor of target protein from the first fusion protein obtained in the first step and further cleaving the second precursor of target protein from the second fusion protein obtained in the second step, both by a cleaving function of the protein consisting of a partial sequence of an intein and the protein consisting of a remaining partial sequence of the intein; and
a fourth step of assembling the first precursor of target protein and the second precursor of target protein both obtained in the third step by an assembling function of the protein consisting of a partial sequence of an intein and the protein consisting of a remaining partial sequence of the intein.

40. A method of producing a target protein, the method comprising steps of:
cleaving a first precursor and a second precursor of target protein from a fusion protein composed of a molecular chaperone, a protein consisting of a partial sequence of an intein linked to one terminus of the molecular chaperone and a protein consisting of a remaining partial sequence of the intein linked to the other terminus of the molecular chaperone both with a peptide bond, and further the first precursor of target protein linked to the protein consisting of a partial sequence of an intein and the second precursor of target protein linked to the protein consisting of a remaining sequence of the intein both with a peptide bond by a cleaving function of the proteins consisting of a partial sequence of intein and the protein consisting of a remaining partial sequence of the intein; and
assembling the first precursor of target protein and the second precursor of target protein by an assembling function of the protein consisting of a partial sequence of an intein and the protein consisting of a remaining partial sequence of the intein.

41. The method as defined in claim 39 or 40,
wherein the fusion protein is expressed in a host.

42. The method as defined in claim 41,
the host being one selected from a group consisting of bacteria, yeasts, animal cells, plant cells, insect cells, animals, plants, and insects.

43. The method as defined in claim 39 or 40,
being performed in a cell-free translation system.


SEQUENCE LISTING

<110>  Sekisui Chemical Co., Ltd.

<120>  METHOD OF PRODUCING TARGET PROTEIN, FUSED PROTEIN AND GENE THEREOF,
       PARTIAL SEQUENCE PROTEIN OF INTEIN AND GENE THEREOF, EXPRESSION VECTOR,
       AND TRANSFORMANT

<130>  471-3

<140>  EP 04 730 018.1
<141>  2004-04-28

<150>  PCT/JP2004/006191
<151>  2004-04-28

<150>  JP2003-124354
<151>  2003-04-28

<150>  JP2003-124355
<151>  2003-04-28

<150>  JP2003-421442
<151>  2003-12-18

<150>  JP2003-421443
<151>  2003-12-18

<160>  41

<170>  PatentIn version 3.1

<210>  1
<211>  30
<212>  DNA
<213>  Artificial

<220>
<223>  Designed oligonucleotide primer for PCR

<400>  1
gaaggatccg ctatctctgg cgatagtctg                                        30


<210>  2
<211>  33
<212>  DNA
<213>  Artificial

<220>
<223>  Designed oligonucleotide primer for PCR

<400>  2
aggtgatcag ttgtgtacaa tgatgtcatt cgc                                    33


<210>  3
<211>  462
<212>  DNA
<213>  Synechocystis PCC6803

<220>
<221>  CDS
<222>  (1)..(462)
<223>

<400>  3
gct atc tct ggc gat agt ctg atc agc ctg gct agc aca gga aaa aga         48
Ala Ile Ser Gly Asp Ser Leu Ile Ser Leu Ala Ser Thr Gly Lys Arg

1                   5                   10                  15

gtt tct att aaa gat ttg tta gat gaa aaa gat ttt gaa ata tgg gca        96
Val Ser Ile Lys Asp Leu Leu Asp Glu Lys Asp Phe Glu Ile Trp Ala
            20                  25                  30

att aat gaa cag acg atg aag cta gaa tca gct aaa gtt agt cgt gta       144
Ile Asn Glu Gln Thr Met Lys Leu Glu Ser Ala Lys Val Ser Arg Val
        35                  40                  45

ttt tgt act ggc aaa aag cta gtt tat att cta aaa act cga cta ggt       192
Phe Cys Thr Gly Lys Lys Leu Val Tyr Ile Leu Lys Thr Arg Leu Gly
    50                  55                  60

aga act atc aag gca aca gca aat cat aga ttt tta act att gat ggt       240
Arg Thr Ile Lys Ala Thr Ala Asn His Arg Phe Leu Thr Ile Asp Gly
65                  70                  75                  80

tgg aaa aga tta gat gag cta tct tta aaa gag cat att gct cta ccc       288
Trp Lys Arg Leu Asp Glu Leu Ser Leu Lys Glu His Ile Ala Leu Pro
                85                  90                  95

cgt aaa cta gaa agc tcc tct tta caa ttg tca cca gaa ata gaa aag       336
Arg Lys Leu Glu Ser Ser Ser Leu Gln Leu Ser Pro Glu Ile Glu Lys
            100                 105                 110

ttg tct cag agt gat att tac tgg gac tcc atc gtt tct att acg gag       384
Leu Ser Gln Ser Asp Ile Tyr Trp Asp Ser Ile Val Ser Ile Thr Glu
        115                 120                 125

act gga gtc gaa gag gtt ttt gat ttg act gtg cca gga cca cat aac       432
Thr Gly Val Glu Glu Val Phe Asp Leu Thr Val Pro Gly Pro His Asn
    130                 135                 140

ttt gtc gcg aat gac atc att gta cac aac                               462
Phe Val Ala Asn Asp Ile Ile Val His Asn
145                 150

<210>  4
<211>  154
<212>  PRT
<213>  Synechocystis PCC6803

<400>  4

Ala Ile Ser Gly Asp Ser Leu Ile Ser Leu Ala Ser Thr Gly Lys Arg
1               5                   10                  15

Val Ser Ile Lys Asp Leu Leu Asp Glu Lys Asp Phe Glu Ile Trp Ala
            20                  25                  30

Ile Asn Glu Gln Thr Met Lys Leu Glu Ser Ala Lys Val Ser Arg Val
        35                  40                  45

Phe Cys Thr Gly Lys Lys Leu Val Tyr Ile Leu Lys Thr Arg Leu Gly
    50                  55                  60

Arg Thr Ile Lys Ala Thr Ala Asn His Arg Phe Leu Thr Ile Asp Gly
65                  70                  75                  80

Trp Lys Arg Leu Asp Glu Leu Ser Leu Lys Glu His Ile Ala Leu Pro
                85                  90                  95

Arg Lys Leu Glu Ser Ser Ser Leu Gln Leu Ser Pro Glu Ile Glu Lys
            100                 105                 110

Leu Ser Gln Ser Asp Ile Tyr Trp Asp Ser Ile Val Ser Ile Thr Glu
        115                 120                 125

Thr Gly Val Glu Glu Val Phe Asp Leu Thr Val Pro Gly Pro His Asn
    130                 135                 140

Phe Val Ala Asn Asp Ile Ile Val His Asn
145                 150

<210>  5
<211>  33
<212>  DNA
<213>  Artificial

<220>
<223>  Designed oligonucleotide primer for PCR

<400>  5
aggagatcta gtgcccagct tgcaggccag cca                                    33

<210>  6
<211>  33
<212>  DNA
<213>  Artificial

<220>
<223>  Designed oligonucleotide primer for PCR

<400>  6
gaaggatcct cagtcgagat cgcttccgaa gtc                                    33

<210>  7
<211>  1641
<212>  DNA
<213>  Thermococcus sp.

<400>  7
atggcccagc ttgcaggcca gccagttgtt attctacctg agggaactca gaggtacgtt      60

ggaagggacg cccagaggct caacattctt gctgccagga ttatagccga gacggttaga     120

accacccttg gaccaaaggg aatggacaag atgctcgttg acagcctcgg cgacatcgtc     180

atcaccaacg acggtgcaac cattctcgac gagatggaca tccagcaccc tgctgctaag     240

atgatggttg aggttgctaa gactcaggat aaggaggctg gtgatggtac tactactgcg     300

gttgttattg ctggtgagct tctgaggaag gctgaggagc ttctcgacca gaacattcac     360

ccgagcataa tcatcaaggg ctacgccctc gcagcagaga aagcccagga aatactcgac     420

gagatagcca aggacgttga cgtcgaggac agggagattc tcaagaaggc cgcggtcacc     480

tccatcaccg gaaaggccgc cgaggaggag agggagtacc tcgctgagat agcagttgag     540

gccgtcaagc aggttgccga gaaggttggc gagacctaca aggtcgacct cgacaacatc     600

aagttcgaga agaaggaagg tggaagcgtc aaggacaccc agctcataaa gggtgtcgtc     660

atcgacaagg aggtcgtcca cccaggcatg ccgaagaggg tcgagggtgc taagatcgcc 720

ctcatcaacg aggcccttga ggtcaaggag actgagaccg acgccgagat caggatcacc 780

agcccggagc agctccaggc cttccttgag caggaggaga agatgctcag ggagatggtc 840

gacaagatca aggaggtcgg cgcgaacgtc gtgttcgtcc agaagggcat tgacgacctt 900

gcccagcact acctggccaa gtacggcata atggcagtca ggagggtcaa gaagagcgac 960

atggagaagc tcgccaaggc cactggagct aagatcgtca ccaacgtccg cgacctcacc 1020

ccggaggacc tcggtgaggc cgagctcgtc gagcagagga aggtcgccgg cgagaacatg 1080

atcttcgtcg agggctgcaa gaacccgaag gcagtgacaa tactcatcag gggcggtacc 1140

gagcacgtcg ttgacgaggt cgagagggcc ctcgaggatg ccgtcaaggt cgtcaaggac 1200

atcgtcgagg acggcaagat cgtcgccgcc ggcggtgctc cggagatcga gctcagcatc 1260

aggctcgacg agtacgcgaa ggaggtcggc ggcaaggagc agctcgccat cgaggccttt 1320

gcagaggccc tcaaggtcat tccgaggacc ctcgccgaga acgccggtct cgacccgatc 1380

gagaccctcg ttaaggtcat cgccgcccac aaggagaagg gaccgaccat cggtgttgac 1440

gtcttcgagg gcgagccggc cgacatgctc gagcgcggcg tcatcgcccc ggtcagggtt 1500

ccgaagcagg ccatcaagag cgccagcgag gccgccataa tgatcctcag gatcgacgac 1560

gtcatcgccg ccagcaagct cgagaaggac aaggagggcg gcaagggcgg tagcgaggac 1620

ttcggaagcg atctcgactg a 1641

<210> 8
<211> 6
<212> PRT
<213> Artificial

<220>
<223> HIs-Tag peptide

<400> 8

His His His His His His
1               5

<210> 9
<211> 711
<212> DNA
<213> Homo sapiens

<400> 9
atggaaaccc cagcgcagct tctcttcctc ctgctactct ggctcccagt ttcagatacc 60

actggagaaa tagtgatgac gcagtctcca gccaccctgt ctgtgtctcc aggggaaaga 120

gccaccctct cctgcagggc cagtcagagt gttagaagca acttagcctg gtaccagcag 180

aaacctggcc aggctcccag gctcctcatc tatgctgcat ccaccagggc cactggtatc 240

ccagccaggt tcagtggcag tgggtctggg acagagttca ctctcaccat cagcagcctg 300

cagtctgaag atttcgcagt ttattactgt cagcagtata atgaatggtt tcggacgttc 360

ggccaaggga ccaaggtgga aatcaaacga actgtggctg caccatctgt cttcatcttc 420

ccgccatctg atgagcagtt gaaatctgga actgcctctg ttgtgtgcct gctgaataac 480

ttctatccca gagaggccaa agtacagtgg aaggtggata acgccctcca atcgggtaac 540

tcccaggaga gtgtcacaga gcaggacagc aaggacagca cctacagcct cagcagcacc 600

ctgacgctga gcaaagcaga ctacgagaaa cacaaagtct acgcctgcga agtcacccat 660

cagggcctga gctcgcccgt cacaaagagc ttcaacaggg gagagtgtta g 711

<210> 10
<211> 24
<212> DNA
<213> Artificial

<220>
<223> Designed oligonucleotide linker

<400> 10
ctggaagttc tgttccaggg tccg 24

<210> 11
<211> 576
<212> DNA
<213> Hepatitis C virus

<400> 11
atgtctacta acccgaaacc gcagcgtaaa actaaacgta acactaaccg tcgcccacag 60

gacgtcaagt tccctggtgg tggtcagatc gttggtggcg tttacctgct tccacgccgt 120

ggcccacgtc tgggtgtgcg tgcgactcgt aagacttccg agcgctctca acctcgtggt 180

cgtcgtcaac ctatcccgaa ggctcgtcgt ccagagggtc gtgcctgggc tcagccaggt 240

tacccttggc cactctatgg caatgagggc atgggttggg caggttggct cctgtctcca 300

cgcggttccc gtcctagctg gggtccgact gacccacgtc gtcgctctcg taacctgggt 360

aaggtcatcg ataccctcac atgcggcttc gccgacctca tgggttacat tccgctcgtc 420

ggtgccccac tgggtggtgc tgcccgtgcc ctggcgcatg gcgtccgtgt tctggaagac 480

ggcgtgaact atgcaacagg taatctgcca ggttgctctt tctctatctt cctcctggct 540

ctgctgtcct gcctgaccat cccagcctcc gcttaa 576

<210> 12
<211> 35
<212> DNA
<213> Artificial

<220>
<223> Designed oligonucleotide primer for PCR

<400> 12
aagtctagat gtatctctgg cgatagtctg atcag 35

<210> 13
<211> 36
<212> DNA
<213> Artificial

<220>
<223> Designed oligonucleotide primer for PCR

<400>  13
aagagatcta gcgtgtacaa tgatgtcatt cgcgac                                36

<210>  14
<211>  462
<212>  DNA
<213>  Synechocystis PCC6803

<400>  14
tgtatctctg gcgatagtct gatcagcctg gctagcacag gaaaaagagt ttctattaaa      60

gatttgttag atgaaaaaga ttttgaaata tgggcaatta atgaacagac gatgaagcta     120

gaatcagcta aagttagtcg tgtattttgt actggcaaaa agctagttta tattctaaaa     180

actcgactag gtagaactat caaggcaaca gcaaatcata gatttttaac tattgatggt     240

tggaaaagat tagatgagct atctttaaaa gagcatattg ctctaccccg taaactagaa     300

agctcctctt tacaattgtc accagaaata gaaaagttgt ctcagagtga tatttactgg     360

gactccatcg tttctattac ggagactgga gtcgaagagg tttttgattt gactgtgcca     420

ggaccacata actttgtcgc gaatgacatc attgtacacg ct                        462

<210>  15
<211>  42
<212>  DNA
<213>  Artificial

<220>
<223>  Designed oligonucleotide primer for PCR

<400>  15
agccactagt gcagctaaag acgtaaaatt cggtaacgac gc                         42

<210>  16
<211>  30
<212>  DNA
<213>  Artificial

<220>
<223>  Designed oligonucleotide primer for PCR

<400>  16
tgcctctaga caggtcggta accatgcatt                                       30

<210>  17
<211>  1647
<212>  DNA
<213>  Escherichia coli

<400>  17
atggcagcta aagacgtaaa attcggtaac gacgctcgtg tgaaaatgct gcgcggcgta      60

aacgtactgg cagatgcagt gaaagttacc ctcggtccaa aaggccgtaa cgtagttctg     120

gataaatctt tcggtgcacc gaccatcacc aaagatggtg tttccgttgc tcgtgaaatc     180

gaactggaag acaagttcga aaatatgggt gcgcagatgg tgaaagaagt tgcctctaaa     240

gcaaacgacg ctgcaggcga cggtaccacc actgcaaccg tactggctca ggctatcatc     300

actgaaggtc tgaaagctgt tgctgcgggc atgaacccga tggacctgaa acgtggtatc     360

gacaaagcgg ttaccgctgc agttgaagaa ctgaaagcgc tgtccgtacc atgctctgac 420

tctaaagcga ttgctcaggt tggtaccatc tccgctaact ccgacgaaac cgtaggtaaa 480

ctgatcgctg aagcgatgga caaagtcggt aaagaaggcg ttatcaccgt tgaagacggt 540

accggtctgc aggacgaact ggacgtggtt gaaggtatgc agttcgaccg tggctacctg 600

tctccttact tcatcaacaa gccggaaact ggcgcagtag aactggaaag cccgttcatc 660

ctgctggctg acaagaaaat ctccaacatc cgcgaaatgc tgccggttct ggaagctgtt 720

gccaaagcag gcaaaccgct gctgatcatc gctgaagatg tagaaggcga agcgctggca 780

actctggttg ttaacaccat gcgtggcatc gtgaaagtcg ctgcggttaa agcaccgggc 840

ttcggcgatc gtcgtaaagc tatgctgcag gatatcgcaa ccctgactgg cggtaccgtg 900

atctctgaag agatcggtat ggagctggaa aaagcaaccc tggaagacct gggtcaggct 960

aaacgtgttg tgatcaacaa agacaccacc actatcatcg atggcgtggg tgaagaagct 1020

gcaatccagg gccgtgttgc tcagatccgt cagcagattg aagaagcaac ttctgactac 1080

gaccgtgaaa aactgcagga acgcgtagcg aaactggcag gcggcgttgc agttatcaaa 1140

gtgggtgctg ctaccgaagt tgaaatgaaa gagaaaaaag cacgcgttga agatgccctg 1200

cacgcgaccc gtgctgcggt agaagaaggc gtggttgctg gtggtggtgt tgcgctgatc 1260

cgcgtagcgt ctaaactggc tgacctgcgt ggtcagaacg aagaccagaa cgtgggtatc 1320

aaagttgcac tgcgtgcaat ggaagctccg ctgcgtcaga tcgtattgaa ctgcggcgaa 1380

gaaccgtctg ttgttgctaa caccgttaaa ggcggcgacg gcaactacgg ttacaacgca 1440

gcaaccgaag aatacggcaa catgatcgac atgggtatcc tggatccaac caaagtaact 1500

cgttctgctc tgcagtacgc agcttctgtg gctggcctga tgatcaccac cgaatgcatg 1560

gttaccgacc tgccgaaaaa cgatgcagct gacttaggcg ctgctggcgg tatgggcggc 1620

atgggtggca tgggcggcat gatgtaa 1647

<210> 18
<211> 8
<212> PRT
<213> Artificial

<220>
<223> FLAG peptide

<400> 18

Asp Tyr Lys Asp Asp Asp Asp Lys
1               5

<210> 19
<211> 957
<212> DNA
<213> Homo sapiens

<400> 19
atggatgact ccacggactc ctctgacaat agcctggctc ttacaagtcc ttataagaca 60

tttgaagtgg tgtttattgt cctggtggct ggatccctca gtttggtgac cattatcggg 120

aacatcctag tcatggtttc cattaaagtc aaccgccacc tccagaccgt caacaattac 180

tttttattca gcttggcctg tgctgacctt atcataggtg ttttctccat gaacttgtac 240

accctctaca ctgtgattgg ttactggcct ttgggacctg tggtgtgtga cctttggcta 300

gccctggact atgtggtcag caatgcctca gttatgaatc tgctcatcat cagctttgac 360

aggtacttct gtgtcacaaa acctctgacc tacccagtca agcggaccac aaaaatggca 420

ggtatgatga ttgcagctgc ctgggtcctc tctttcatcc tctgggctcc agccattctc 480

ttctggcagt tcattgtagg ggtgagaact gtggaggatg gggagtgcta cattcagttt 540

ttttccaatg ctgctgtcac ctttggtacg gctattgcag ccttctattt gccagtgatc 600

atcatgactg tgctatattg gcacatatcc cgagccagca agagcaggat aaagaaggac 660

aagaaggagc ctgttgccaa ccaagacccc gtttcccggg aaaagaaagt caccaggaca 720

atcttggcta ttctgttggc tttcatcatc acttgggccc catacaatgt catggtgctc 780

attaacacct tttgtgcacc ttgcatcccc aacactgtgt ggacaattgg ttactggctt 840

tgttacatca acagcactat caaccctgcc tgctatgcac tttgcaatgc caccttcaag 900

aagaccttta aacaccttct catgtgtcat tataagaaca taggcgctac aaggtaa 957

<210> 20
<211> 33
<212> DNA
<213> Artificial

<220>
<223> Designed oligonucleotide primer for PCR

<400> 20
aagccatgga tgaaagttga agctggtgat tat 33

<210> 21
<211> 33
<212> DNA
<213> Artificial

<220>
<223> Designed oligonucleotide primer for PCR

<400> 21
aagactagtc taagcttctg agtcctcttc ggc 33

<210> 22
<211> 480
<212> DNA
<213> Thermococcus sp.

<400> 22
atgaaagttg aagctggtga ttatgttctc ttccactacg ttggaaggtt cgaggatgga 60

gaagtttttg acacaagcta cgaggagata gccagagaga atggcattct cgtcgaggag 120

agggagtacg gcccaatgtg ggtcaggata ggcgtcggtg agatcatccc tggcctcgat 180

gaagccataa ttggcatgga agctggagag aagaagaccg tgaccgttcc ccccgagaag 240

gcttacggaa tgccgaaccc agagcttgta atctccgttc caagggaaga attcacaaag 300

gccggccttg aaccccagga aggtctctac gtcatgaccg attctggcat agccaagata 360

gtttccgttg gagagagcga ggtatccctt gacttcaacc acccgctagc aggtaagacc 420

ctagtctttg aggtagaagt catagaagta aaaaaggccg aagaggactc agaagcttag 480

<210> 23
<211> 37
<212> DNA
<213> Artificial

<220>
<223> Designed oligonucleotide primer for PCR

<400> 23
aagactagta tgagtaaagg agaagaactt ttcactg 37

<210> 24
<211> 39
<212> DNA
<213> Artificial

<220>
<223> Designed oligonucleotide primer for PCR

<400> 24
aaggttaact ttgtatagtt catccatgcc atgtgtaat 39

<210> 25
<211> 717
<212> DNA
<213> Aquorea victoria

<400> 25
atgagtaaag gagaagaact tttcactgga gttgtcccaa ttcttgttga attagatggt 60

gatgttaatg ggcacaaatt ttctgtcagt ggagagggtg aaggtgatgc aacatacgga 120

aaacttaccc ttaaatttat ttgcactact ggaaaactac ctgttccatg gccaacactt 180

gtcactactt tctcttatgg tgttcaatgc ttttcaagat acccagatca tatgaaacag 240

catgactttt tcaagagtgc catgcccgaa ggttatgtac aggaaagaac tatatttttc 300

aaagatgacg ggaactacaa gacacgtgct gaagtcaagt ttgaaggtga tacccttgtt 360

aatagaatcg agttaaaagg tattgatttt aaagaagatg gaaacattct tggacacaaa 420

ttggaataca actataactc acacaatgta tacatcatgg cagacaaaca aaagaatgga 480

atcaaagtta acttcaaaat tagacacaac attgaagatg gaagcgttca actagcagac 540

cattatcaac aaaatactcc aattggcgat ggccctgtcc ttttaccaga caaccattac 600

ctgtccacac aatctgccct ttcgaaagat cccaacgaaa agagagacca catggtcctt 660

cttgagtttg taacagctgc tgggattaca catggcatgg atgaactata caaataa 717

<210> 26
<211> 35
<212> DNA
<213> Artificial

<220>

<223>  Designed oligonucleotide primer for PCR

<400>  26
aagagatcta tggaaaccct ttgcctcagg gcatc                                35


<210>  27
<211>  38
<212>  DNA
<213>  Artificial

<220>
<223>  Designed oligonucleotide primer for PCR

<400>  27
aaggagctct cagttcatgc tgtccttatg gctgctcc                             38


<210>  28
<211>  1284
<212>  DNA
<213>  Homo sapiens

<400>  28
atggaaaccc tttgcctcag ggcatccttt tggctggcac tggttggatg tgtaatcagt      60

gataatcctg agagatacag cacaaatcta agcaatcatg tggatgattt caccactttt     120

cgtggcacag agctcagctt cctggttacc actcatcaac ccactaattt ggtcctaccc     180

agcaatggct caatgcacaa ctattgccca cagcagacta aaattacttc agctttcaaa     240

tacattaaca ctgtgatatc ttgtactatt ttcatcgtgg gaatggtggg gaatgcaact     300

ctgctcagga tcatttacca gaacaaatgt atgaggaatg gccccaacgc gctgatagcc     360

agtcttgccc ttggagacct tatctatgtg gtcattgatc tccctatcaa tgtatttaag     420

ctgctggctg ggcgctggcc ttttgatcac aatgactttg gcgtatttct ttgcaagctg     480

ttcccctttt tgcagaagtc ctcggtgggg atcaccgtcc tcaacctctg cgctcttagt     540

gttgacaggt acagagcagt tgcctcctgg agtcgtgttc agggaattgg gattcctttg     600

gtaactgcca ttgaaattgt ctccatctgg atcctgtcct ttatcctggc cattcctgaa     660

gcgattggct tcgtcatggt accctttgaa tataggggtg aacagcataa aacctgtatg     720

ctcaatgcca catcaaaatt catggagttc taccaagatg taaaggactg gtggctcttc     780

gggttctatt tctgtatgcc cttggtgtgc actgcgatct tctacaccct catgacttgt     840

gagatgttga acagaaggaa tggcagcttg agaattgccc tcagtgaaca tcttaagcag     900

cgtcgagaag tggcaaaaac agttttctgc ttggttgtaa tttttgctct ttgctggttc     960

cctcttcatt taagccgtat attgaagaaa actgtgtata acgagatgga caagaaccga    1020

tgtgaattac ttagtttctt actgctcatg gattacatcg gtattaactt ggcaaccatg    1080

aattcatgta taaaccccat agctctgtat tttgtgagca agaaatttaa aaattgtttc    1140

cagtcatgcc tctgctgctg ctgttaccag tccaaaagtc tgatgacctc ggtccccatg    1200

aacggaacaa gcatccagtg gaagaaccac gatcaaaaca accacaacac agaccggagc    1260

agccataagg acagcatgaa ctga                                           1284

<210>  29
<211>  36
<212>  DNA
<213>  Artificial

<220>
<223>  Designed oligonucleotide primer for PCR

<400>  29
gaaggatccc cagaaataga aaagttgtct cagagt                                36


<210>  30
<211>  33
<212>  DNA
<213>  Artificial

<220>
<223>  Designed oligonucleotide primer for PCR

<400>  30
gaaactagtg ttgtgtacaa tgatgtcatt cgc                                   33


<210>  31
<211>  144
<212>  DNA
<213>  Synechocystis PCC6803

<220>
<221>  CDS
<222>  (1)..(144)
<223>

<400>  31
tca cca gaa ata gaa aag ttg tct cag agt gat att tac tgg gac tcc        48
Ser Pro Glu Ile Glu Lys Leu Ser Gln Ser Asp Ile Tyr Trp Asp Ser
1               5                   10                  15

atc gtt tct att acg gag act gga gtc gaa gag gtt ttt gat ttg act        96
Ile Val Ser Ile Thr Glu Thr Gly Val Glu Glu Val Phe Asp Leu Thr
            20                  25                  30

gtg cca gga cca cat aac ttt gtc gcg aat gac atc att gta cac aac       144
Val Pro Gly Pro His Asn Phe Val Ala Asn Asp Ile Ile Val His Asn
        35                  40                  45


<210>  32
<211>  48
<212>  PRT
<213>  Synechocystis PCC6803

<400>  32

Ser Pro Glu Ile Glu Lys Leu Ser Gln Ser Asp Ile Tyr Trp Asp Ser
1               5                   10                  15


Ile Val Ser Ile Thr Glu Thr Gly Val Glu Glu Val Phe Asp Leu Thr
            20                  25                  30


Val Pro Gly Pro His Asn Phe Val Ala Asn Asp Ile Ile Val His Asn
        35                  40                  45


<210>  33
<211>  1401

<212>  DNA
<213>  Homo sapiens

<400>  33
atggagtttg ggctgagctg ggttttcctc gttgctcttt taagaggtgt ccagtgtcag      60

gtgcagctgg tggagtctgg gggaggcgtg gtccagcctg ggaggtccct gagactctcc     120

tgtgtagcct ctggattcac cttcagtagc tatcctatga cctgggtcca gccagctcca     180

ggcaaggggc tggagtgggt ggcaagtata tcatatgacg gaagttataa atataaggta     240

gactccatga agggccgact caccatctcc agagacaatt ccaagaacac gctgtatttg     300

gaaatgaaca gcctgacagc tgaggacacg gctgtgtatt actgtgcgag gacagcttgc     360

tttaacgcct atgacttctg gggccaggga accctggtca ccgtctcctc agcctccacc     420

aagggcccat cggtcttccc cctggcaccc tcctccaaga gcacctctgg gggcacagcg     480

gccctgggct gcctggtcaa ggactacttc cccgaaccgg tgacggtgtc gtggaactca     540

ggcgccctga ccagcggcgt gcacaccttc ccggctgtcc tacagtcctc aggactctac     600

tccctcagca gcgtggtgac cgtgccctcc agcagcttgg gcacccagac ctacatctgc     660

aacgtgaatc acaagcccag caacaccaag gtggacaaga aagttgagcc caaatcttgt     720

gacaaaactc acacatgccc accgtgccca gcacctgaac tcctgggggg accgtcagtc     780

ttcctcttcc ccccaaaacc caaggacacc ctcatgatct cccggacccc tgaggtcaca     840

tgcgtggtgg tggacgtgag ccacgaagac cctgaggtca agttcaactg gtacgtggac     900

ggcgtggagg tgcataatgt taagacaaag ccgcgggagg agcagtacaa cagcacgtac     960

cgtgtggtca gcgtcctcac cgtcctgcac cagaactgga tgaatggaaa ggagtacaag    1020

tgcaaggtct ccaacaaagc cctcccagcc cccatcgaga aaaccatctc caaagccaaa    1080

gtgcagcccc gagaaccaca ggtgtacacc ctgcccccat cccgggatga gctgaccaag    1140

aaccaggtca gcctgacctg cctggtcaaa ggcttctatc ccagcgacat cgccgtggag    1200

tgggagagca atgggcagcc ggagaacaac tacaagacca cgcctcccgt gctggactcc    1260

gtcggctcct tcttcctcta cagcaagctc accgtggaca agagcaggtg gcagcagggg    1320

aacgtcttct catgctccgt gatgcatgag gctctgcaca accactacca gcagaggagc    1380

ctctccctgt ctccgggtaa a                                              1401

<210>  34
<211>  43
<212>  DNA
<213>  Artificial

<220>
<223>  Designed oligonucleotide linker

<400>  34
gatccactag tgttacccac caccaccacc accactaata ggc                        43

<210>  35
<211>  40
<212>  DNA
<213>  Artificial

<220>
<223>  Designed oligonucleotide linker

<400>  35
ggactagtct ggttccgcgt ggatcccata tggaatccgg                                40

<210>  36
<211>  50
<212>  DNA
<213>  Artificial

<220>
<223>  Designed oligonucleotide linker

<400>  36
catggccgga tccactagtg ttacccacca ccaccaccac cactaatagg                     50

<210>  37
<211>  52
<212>  DNA
<213>  Artificial

<220>
<223>  Designed oligonucleotide linker

<400>  37
cggccgccta ttagtggtgg tggtggtggt gggtaacact agaggatccg gc                  52

<210>  38
<211>  56
<212>  DNA
<213>  Artificial

<220>
<223>  Designed oligonucleotide linker

<400>  38
catggcctct agaagatctc tcgaggacta caaagacgac gacgacaaat aataga              56

<210>  39
<211>  56
<212>  DNA
<213>  Artificial

<220>
<223>  Designed oligonucleotide linker

<400>  39
agcttctatt atttgtcgtc gtcgtctttg tagtcctcga gagatcttct agtggc              56

<210>  40
<211>  27
<212>  DNA
<213>  Artificial

<220>
<223>  Designed oligonucleotide linker

<400>  40
catgagccac caccaccacc accacgg                                              27

<210>  41

<211>  27
<212>  DNA
<213>  Artificial

<220>
<223>  Designed oligonucleotide linker

<400>  41
catgccgtgg tggtggtggt ggtggct                                          27